# EUROPEAN PATENT APPLICATION

(11) **EP 4 487 808 A1**
(43) Date of publication of application: **08.01.2025**
(21) Application number: 23762789.8
(22) Date of filing: 22.02.2023
(51) Int. Cl.: A61B 34/30

(54) **POWER APPARATUS, SURGICAL ROBOT AND JOINT METHOD**

(30) Priority: 04.03.2022 CN 202210213585; 04.03.2022 CN 202210212330
(71) Applicant: Shenzhen Edge Medical Co., Ltd., Shenzhen, Guangdong 518000 (CN)
(72) Inventor: LIU, Fang, Shenzhen, Guangdong 518000 (CN); LIN, Mincai, Shenzhen, Guangdong 518000 (CN); SUN, Qiang, Shenzhen, Guangdong 518000 (CN); WANG, Jianchen, Shenzhen, Guangdong 518000 (CN)
(74) Representative: Biallo, Dario
(86) International application number: PCT/CN2023/077630
(87) International publication number: WO 2023/165389

(57) **Abstract**

Apower apparatus (100) for a surgical robot, a surgical robot and a joint method, and a detecting method. The power apparatus (100) comprises: a rotating component (10), an axial biasing component (20) for providing an elastic bias for the rotating component (10), a first detection component (30), a second detection component (60), and a controller (40). Before a driving input interface (50) is mounted, the controller (40) rotates the rotating component (10) until a target portion (T) is detected by the second detection component (60), and when the detected portion of the rotating component (10) is located on the side facing away from the surgical instrument (200) relative to a preset position (A0), the controller (40) rotates, according to a detection result of the first detection component (30), the rotating component (10) in a preset manner to enable the rotating component (10) to move along an axial direction thereof to the preset position (A0). By using the first detection component (30) to detect the position of the rotating component (10) for outputting torque in the axial direction, after the surgical instrument (200) is mounted, the driving coupling state of the power apparatus (100) can be accurately determined according to the detection result of the first detection component (30). Thus, the expected actions can be accurately executed according to the instruction of the doctor, and the surgical risk caused by an unexpected swing of the instrument joint is avoided.

## Description

### CROSS-REFERENCE TO RELATED APPLICATION

This application claims the priority of Chinese Patent Application No. 202210213585.X, filed on March 4, 2022, and titled "power apparatus, surgical robot, and detecting method" and Chinese Patent Application No. 202210212330.1, filed on March 4, 2022, and titled "power apparatus, surgical robot, and engaging method", the entirety of which are incorporated by reference herein.

### TECHNICAL FIELD

The subject matter herein generally relates to robots, in particular to a power apparatus, a surgical robot, and an engaging method.

### BACKGROUND

Minimally invasive surgery refers to a surgical technique that utilizes modern medical instruments such as laparoscopes, thoracoscopes, and related equipment to perform operations inside the human body. Compared to traditional surgical methods, minimally invasive surgery offers advantages such as smaller incisions, less pain, and faster recovery.

With the development of minimally invasive surgical techniques and artificial intelligence technology, surgical robot now possess the capabilities to perform complex surgeries, offering high stability in surgical outcomes, precise operations, minimal bleeding, and reduced postoperative complications. Robot-assisted minimally invasive surgery is gradually becoming one of the trends in the development of minimally invasive surgical procedures.

Current minimally invasive surgical robot can operate corresponding power apparatus based on the surgeon's manipulations, driving the corresponding surgical instrument to perform surgical operations. However, considering the need for sterilization of surgical instrument, the instrument is typically mounted to a detachable manner onto the main body of the equipment where the power apparatus is located, utilizing the surgical instrument to receive torque from the power apparatus. In the existing mounting process of surgical robot, after installing the surgical instrument or sterile adapter, methods such as encoders etc. can only be used to determine the rotation angle of the motor, and there is no way to judge whether the surgical instrument or sterile adapter is already coupled with the power apparatus. This results in the surgical instrument being unable to accurately complete preset movements based on the surgeon's manipulations, and unexpected swing of the instrument joint caused by the self-checking process when the surgical instrument is not coupled may also pose surgical risk.

### SUMMARY OF THE INVENTION

In view of the deficiencies of related art, the present disclosure provides a power apparatus, a surgical robot, and an engaging method that can accurately identify the drive-coupling state between the power apparatus and surgical instrument, thereby precisely executing the surgeon's instructions and avoiding unexpected swings of the instrument joints.

Aiming at the aforementioned technical problems, the present disclosure provides a power apparatus of a surgical robot. The power apparatus comprises a first housing; a rotating component rotatably provided on the first housing, comprising a target portion and an axial coupling end, wherein the coupling end is configured to be coupled with a drive-input interface providing torque for a surgical instrument; an axial biasing component configured to provide an elastic bias towards the surgical instrument for the rotating component; a first detection component configured to detect a position of the rotating component in an axial direction of the rotating component; a second detection component configured to be aligned with the target portion when the rotating component is rotated to a zero position; and a controller configured to: rotate the rotating component until the target portion is detected by the second detection component before mounting the drive-input interface; and when a detected portion of the rotating component is located at a position further from the surgical instrument than a preset position, rotate the rotating component in a preset manner so as to move the rotating component along the axial direction of the rotating component to the preset position..

In an embodiment, the drive-input interface comprises a first input disk provided on the surgical instrument, and the first input disk and the rotating component are coupled by engagement of concave and convex features on end surfaces facing each other; the controller is configured to: determine whether the first input disk is coupled with the rotating component based on a detection result from the first detection component after the surgical instrument is mounted to the power apparatus.

In an embodiment, the power apparatus further comprises an adapter assembly, wherein the adapter assembly comprises a second housing detachably mounted to the first housing and a second input disk rotatably provided on the second housing; the drive-input interface comprises the second input disk, and the second input disk is capable of being coupled with the rotating component by engagement of concave and convex features on end surfaces facing each other to transmit torque between the rotating component and the surgical instrument; the controller is configured to: determine whether the second input disk is coupled with the rotating component based on a detection result from the first detection component after the second housing is mounted to the power apparatus.

In an embodiment, he second input disk and the first input disk provided on the surgical instrument are coupled by engagement of concave and convex features on end surfaces facing each other, and the second input disk has a freedom along an axial direction of the second housing; the controller is configured to: determine whether the first input disk is coupled with the second input disk based on a detection result from the first detection component after the second input disk is coupled with the rotating component and the surgical instrument is mounted to the power apparatus.

In an embodiment, a detection end of the first detection component is provided on a side of the rotating component that is further from the surgical instrument, and there is a gap along an axial direction of the rotating component between the detection end of the first detection component and the rotating component.

In an embodiment, the first detection component is configured to detect whether the detected portion of the rotating component is sensed; the controller is configured to: determine that the drive-input interface is not coupled with the rotating component when the first detection component senses the detected portion of the rotating component.

In an embodiment, the first detection component is configured to detect a distance between the first detection component and the detected portion; the controller is configured to: determine that the drive-input interface is not coupled with the rotating component when the first detection component detects that the distance between the first detection component and the rotating component is less than a preset distance.

In an embodiment, the power apparatus further comprises an adapter assembly detachably mounted to the first housing, wherein the adapter assembly is configured to transmit torque between the rotating component and the surgical instrument; the controller is configured to: control the rotating component to be rotated until the target portion is detected by the second detection component when the adapter assembly is mounted to the first housing and the first detection component detects that the adapter assembly is drivingly engaged with the rotating component.

In an embodiment, the power apparatus further comprises an adapter assembly, wherein the adapter assembly comprises a second input disk, and the surgical instrument comprises a first input disk coupled with the rotating component via the second input disk; the controller is configured to: control the rotating component to be rotated in a preset first manner when the second input disk is mounted to the rotating component and not coupled with the rotating component, and interrupt rotation of the rotating component in the preset first manner when the rotating component is rotated to be coupled with the second input disk; and/or control the rotating component to be rotated in a preset second manner when the rotating component is coupled with the second input disk and the first input disk is mounted to the second input disk and not coupled with the rotating component, and interrupt rotation of the rotating component in the preset second manner when the rotating component is rotated to be coupled with the first input disk.

In an embodiment, the rotating component comprises a first rotating component and a second rotating component each corresponding to one of the second input disk and one of the first input disk; the controller is configured to: control the first rotating component to be rotated in a preset third manner when the first rotating component is coupled with the second input disk corresponding to the first rotating component and the first input disk corresponding to the first rotating component is mounted to the second input disk corresponding to the first rotating component and not coupled with the first rotating component, interrupt the rotation of the first rotating component in the preset third manner when the first rotating component is rotated to be coupled with the first input disk corresponding to the first rotating component, and control the first rotating component to be rotated until the target portion is detected by the second detection component; and control the second rotating component to be rotated in a preset fourth manner when the second rotating component is coupled with the second input disk corresponding to the second rotating component and the first input disk corresponding to the second rotating component is mounted to the second input disk corresponding to the second rotating component and not coupled with the second rotating component, interrupt the rotation of the second rotating component in the preset fourth manner when the second rotating component is rotated to be coupled with the first input disk corresponding to the second rotating component, and control the second rotating component to be rotated until the target portion is in a preset angular range.

In an embodiment, the rotating component comprises a first rotating component and a second rotating component each corresponding to one of the second input disk and one of the first input disk; the controller is configured to: control the first rotating component to be rotated in a preset third manner when the first rotating component is coupled with the second input disk corresponding to the first rotating component and the first input disk corresponding to the first rotating component is mounted to the second input disk corresponding to the first rotating component and not coupled with the first rotating component, interrupt the rotation of the first rotating component in the preset third manner when the first rotating component is rotated to be coupled with the first input disk corresponding to the first rotating component, and stop rotating the first rotating component; and control the second rotating component to be rotated in a preset fourth manner when the second rotating component is coupled with the second input disk corresponding to the second rotating component and the first input disk corresponding to the second rotating component is mounted to the second input disk corresponding to the second rotating component and not coupled with the second rotating component, interrupt the rotation of the second rotating component in the preset fourth manner when the second rotating component is rotated to be coupled with the first input disk corresponding to the second rotating component, and control the second rotating component to be rotated until the target portion is in a preset angular range.

In an embodiment, the preset first manner and the preset second manner both comprise scanning by rotation in forward and reverse directions, an angle of unidirectional scanning in the first manner is equal to or less than a first angular threshold, the angle of unidirectional scanning in the second manner is equal to or less than a second angular threshold, and the first angular threshold is greater than the second angular threshold.

In an embodiment, the power apparatus further comprises an indication device, wherein the first housing is provided with a first signal terminal, the adapter assembly comprises a second signal terminal, the surgical instrument comprises a third signal terminal, the indication device is provided on the adapter assembly and electrically connected to the second signal terminal; the second signal terminal is capable of being conductively connected to the first signal terminal after the adapter assembly is mounted to the first housing, and is capable of being conductively connected to the third signal terminal after the surgical instrument is mounted to the adapter assembly; the controller is configured to: switch an indication state of the indication device according to the detection result from the first detection component after the adapter assembly is mounted to the first housing and/or after the surgical instrument is mounted to the adapter assembly.

In an embodiment, an edge of a surface of the second input disk having a concave or convex feature is protruded with multiple first protrusions, a surface of the second housing facing the surface of the second input disk having the concave or convex feature is protruded with multiple second protrusions, and the second protrusions are configured to restrict rotation of the second input disk when each of the first protrusions is embedded between two second protrusions.

Aiming at the aforementioned technical problems, the present disclosure provides a surgical robot. The surgical robot comprises a surgical instrument and the power apparatus according to any one of the aforementioned embodiments. The surgical instrument is configured to perform corresponding actions driven by the power apparatus.

Aiming at the aforementioned technical problems, the present disclosure provides a detecting method for the engagement state of a power apparatus of a surgical robot. The power apparatus comprises a rotating component comprising an axial coupling end; an axial biasing component configured to provide an elastic bias towards a surgical instrument for the rotating component; a first detection component configured to detect a position of the rotating component in an axial direction of the rotating component. The method comprises: determining whether the drive-input interface is coupled with the rotating component based on the detection result from the first detection component, wherein deformation of the axial biasing component is configured to be increased when a drive-input interface providing torque for the surgical instrument is coupled with the rotating component, the drive-input interface is determined to be not coupled with the rotating component when a detected portion of the rotating component is located at a position further from the surgical instrument than a preset position.

In an embodiment, the drive-input interface comprises a first input disk provided on the surgical instrument, and the first input disk and the rotating component are coupled by engagement of concave and convex features on end surfaces facing each other; the method comprise: determining whether the first input disk is coupled with the rotating component based on a detection result from the first detection component after the surgical instrument is mounted to the power apparatus.

In an embodiment, the power apparatus further comprises an adapter assembly, wherein the adapter assembly comprises a second input disk (52) having a axial and a circumferential degrees of freedom, the first input disk and the rotating component are coupled by engagement of concave and convex features on end surfaces facing each other, the second input disk is coupled with a first input disk provided on the surgical instrument by engagement of concave and convex features on end surfaces facing each other; the method comprise: determining whether the first input disk is coupled with the rotating component based on a detection result from the first detection component after the surgical instrument is mounted to the power apparatus; determining whether the second input disk is coupled with the rotating component based on a detection result from the first detection component after the second housing is mounted to the power apparatus.

In an embodiment, the first detection component is configured to detect whether a detected portion of the rotating component is sensed. The method comprises: determining that the drive-input interface is not coupled with the rotating component when the first detection component senses the detected portion of the rotating component.

In an embodiment, the first detection component is configured to detect a distance between the first detection component and the rotating component. The method comprises: determining that the drive-input interface is not coupled with the rotating component when the distance between the first detection component and the rotating component is less than a preset distance.

In an embodiment, the power apparatus further comprises a first signal terminal. The method comprises: determining that the drive-input interface is mounted to the power apparatus based on signal indicating conduction of the first signal terminal and the signal terminal of the drive-input interface.

In an embodiment, the power apparatus further comprises an adapter assembly which is provided with a second signal terminal and transmits torque between the rotating component and the surgical instrument. The method comprises: determining that the adapter assembly is mounted to the power apparatus based on signal indicating conduction of the first signal terminal and the second signal terminal; and/or determining that the surgical instrument is mounted to the power apparatus based on signal indicating conduction of the first signal terminal and the third signal terminal of the surgical instrument after the adapter assembly is mounted to the power apparatus.

In an embodiment, the power apparatus further comprises an indication device and a first signal terminal, the adapter assembly comprises a second signal terminal the surgical instrument comprises a third signal terminal, the indication device is provided on the adapter assembly and electrically connected to the second signal terminal; the second signal terminal is capable of being conductively connected to the first signal terminal after the a adapter assembly is mounted to the power apparatus, and is capable of being conductively connected to the third signal terminal after the surgical instrument is mounted to the adapter assembly. The method comprises: switching an indication state of the indication device according to the detection result from the first detection component after the adapter assembly is mounted to the first housing and/or after the surgical instrument is mounted to the adapter assembly.

Aiming at the aforementioned technical problems, the present disclosure provides an engaging method for a power apparatus of a surgical robot. The power apparatus comprises a rotating component comprising an axial coupling end; an axial biasing component configured to provide an elastic bias towards a surgical instrument for the rotating component, wherein deformation of the axial biasing component is increased when a drive-input interface providing torque for the surgical instrument is coupled with the rotating component; a first detection component configured to detect a position of the rotating component in an axial direction of the rotating component. The engaging method comprises: detecting the position of the rotating component in the axial direction of the rotating component after the drive-input interface is mounted the rotating component of the power apparatus; and rotating the rotating component in a preset manner when a detected portion of the rotating component is located at a position further from the surgical instrument than a preset position so as to move the rotating component along the axial direction of the rotating component to the preset position.

In an embodiment, the power apparatus further comprises a second detection component, and the rotating component is provided with a target portion. The engaging method further comprises rotating the rotating component until the target portion is detected by the second detection component before mounting the drive-input interface.

In an embodiment, the drive-input interface comprises a first input disk provided on the surgical instrument, and the first input disk and the rotating component are coupled by engagement of concave and convex features on end surfaces facing each other.

In an embodiment, the power apparatus further comprises an adapter assembly comprising a second input disk having an axial degree of freedom and a circumferential degree of freedom, the second input disk is capable of being coupled with the rotating component by engagement of concave and convex features on end surfaces facing each other, and the second input disk is coupled with a first input disk provided on the surgical instrument by engagement of concave and convex features on end surfaces facing each other; the engaging method comprises: mounting the adapter assembly to the power apparatus; detecting the position of the rotating component in the axial direction of the rotating component; rotating the rotating component in a preset first manner when the detected portion of the rotating component is located at the position further from the surgical instrument than the preset position so as to move the rotating component along the axial direction of the rotating component to the preset position; mounting the surgical instrument to the adapter assembly; detecting the position of the rotating component in the axial direction of the rotating component; and rotating the rotating component in a preset second manner when the detected portion of the rotating component is located at the position further from the surgical instrument than the preset position so as to move the rotating component along the axial direction of the rotating component to the preset position.

In an embodiment, the power apparatus further comprises a second detection component, and the rotating component is provided with a target portion; the engaging method comprises: controlling rotation of the rotating component until the target portion is detected by the second detection component after the adapter assembly is mounted to the power apparatus and the rotating component is located in the preset position along the axial direction of the rotating component.

In an embodiment, the engaging method further comprises: after rotating the rotating component in a preset first manner, interrupting the rotation of the rotating component in the preset first manner when the rotating component moves to the preset position along the axial direction of the rotating component; and/or after rotating the rotating component in a preset second manner, interrupting the rotation of the rotating component in the preset second manner when the rotating component moves to the preset position along the axial direction of the rotating component.

In an embodiment, the power apparatus further comprises a second detection component, and the rotating component is provided with a target portion; the rotating component comprises a first rotating component and a second rotating component each corresponding to one of the second input disk and one of the first input disk; the engagement method comprises: controlling the first rotating component to be rotated in a preset third manner when the first rotating component is coupled with the second input disk corresponding to the first rotating component and the first input disk corresponding to the first rotating component is mounted to the second input disk corresponding to the first rotating component and not coupled with the first rotating component, interrupt the rotation of the first rotating component in the preset third manner when the first rotating component is rotated to be coupled with the first input disk corresponding to the first rotating component, and control the first rotating component to be rotated until the target portion is detected by the second detection component; and controlling the second rotating component to be rotated in a preset fourth manner when the second rotating component is coupled with the second input disk corresponding to the second rotating component and the first input disk corresponding to the second rotating component is mounted to the second input disk corresponding to the second rotating component and not coupled with the second rotating component, interrupt the rotation of the second rotating component in the preset fourth manner when the second rotating component is rotated to be coupled with the first input disk corresponding to the second rotating component, and control the second rotating component to be rotated until the target portion is in a preset angular range.

In an embodiment, the power apparatus further comprises a second detection component, and the rotating component is provided with a target portion; the rotating component comprises a first rotating component and a second rotating component each corresponding to one of the second input disk and one of the first input disk; the engagement method comprises: controlling the first rotating component to be rotated in a preset third manner when the first rotating component is coupled with the second input disk corresponding to the first rotating component and the first input disk corresponding to the first rotating component is mounted to the second input disk corresponding to the first rotating component and not coupled with the first rotating component, interrupt the rotation of the first rotating component in the preset third manner when the first rotating component is rotated to be coupled with the first input disk corresponding to the first rotating component, and stop rotating the first rotating component; and controlling the second rotating component to be rotated in a preset fourth manner when the second rotating component is coupled with the second input disk corresponding to the second rotating component and the first input disk corresponding to the second rotating component is mounted to the second input disk corresponding to the second rotating component and not coupled with the second rotating component, interrupt the rotation of the second rotating component in the preset fourth manner when the second rotating component is rotated to be coupled with the first input disk corresponding to the second rotating component, and control the second rotating component to be rotated until the target portion is in a preset angular range.

Aiming at the aforementioned technical problems, the present disclosure provides a control method for a surgical robot comprising an operating part and a slave device. The slave device comprises: multiple rotating components each comprising an axial coupling end; multiple axial biasing components each configured to provide an elastic bias toward a surgical instrument for each of the rotating components, wherein deformations of the axial biasing components are increased when drive-input interfaces providing torque for the surgical instrument are coupled with the rotating components; and multiple first detection components, each configured to detect a position of one of the rotating components in an axial direction of the one of the rotating components. The control method comprises: determining whether the drive-input interfaces are coupled with the rotating components based on detection results from the first detection components; and aligning a posture of the operating part with a posture of the slave device after all the drive-input interfaces of the surgical instrument are coupled with corresponding rotating components of the power apparatus.

In an embodiment, the rotating components comprises a first rotating component and a second rotating component each corresponding to a second input disk and a first input disk; before aligning the posture of the operating part with the posture of the slave device and after all the drive-input interfaces of the surgical instrument are coupled with corresponding rotating components of the power apparatus, the control method further comprises: detecting whether the target portion of each of the second rotating components is rotated to be a preset angular range; and aligning the posture of the operating part with the posture of the slave device when the target portion of each of the second rotating component is rotated to be in the preset angular range (α), otherwise, not performing the posture alignment step.

The power apparatus provided by the present disclosure utilize a first detection component to detect the axial position of a rotating component configured to output torque. This allows for accurately determining the drive-coupling state of the power apparatus based on the detection result from the first detection component after the surgical instrument is mounted. Consequently, the intended movements according to the surgeon's instructions can be precisely executed, and surgical risk caused by unexpected swings of the instrument joints can be avoided.

### BRIEF DESCRIPTION OF THE DRAWINGS

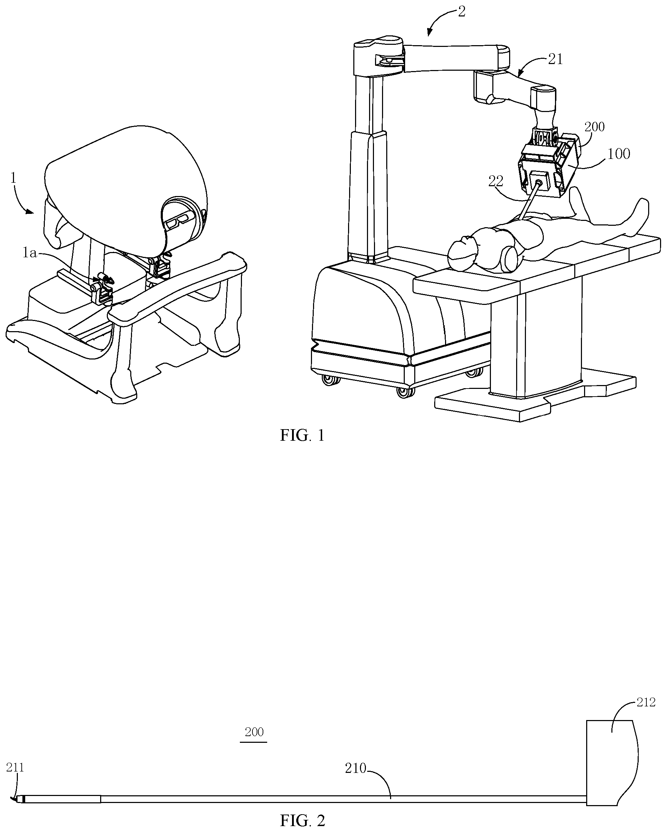

FIG. 1 is a schematic view of a surgical robot according to Embodiment 1 of the present disclosure;
FIG. 2 is a schematic view of a surgical instrument according to Embodiment 1 of the present disclosure;
FIG. 3 is a schematic view of a partial structure of a surgical instrument according to Embodiment 1 of the present disclosure;
FIG. 4A is a schematic view of the internal structure of a power apparatus in a coupled state with a surgical instrument according to Embodiment 1 of the present disclosure;
Fig. 4B is a schematic view of the internal structure of a power apparatus in an uncoupled state with a surgical instrument according to Embodiment 1 of the present disclosure;
Fig. 4C is a schematic view of the internal structure of another power apparatus using a tension spring in a coupled state with a surgical instrument according to Embodiment 1 of the present disclosure;
Fig. 5A is a schematic view of the main structure of a power apparatus according to Embodiment 1 of the present disclosure;
Fig. 5B is a schematic view of the main structure of another power apparatus according to Embodiment 1 of the present disclosure;
Fig. 6A is an exploded view of a power apparatus comprising and adapter assembly according to Embodiment 1 of the present disclosure;
Fig. 6B is a partial cross-sectional view of an adapter assembly according to Embodiment 1 of the present disclosure;
Fig. 7 is a schematic view of an adapter assembly according to Embodiment 1 of the present disclosure;
Fig. 8 is a schematic view of a coupling state with a surgical instrument without an adapter assembly according to Embodiment 1 of the present disclosure;
FIG. 9 is a partial schematic view of another adapter assembly according to Embodiment 1 of the present disclosure;
FIG. 10 is a flow chart of a detecting method for the engagement state of a power apparatus of a surgical robot according to Embodiment 2 of the present disclosure;
Fig. 11 is a flow chart of a method for engaging a power apparatus of a surgical robot according to Embodiment 3 of the present disclosure;
FIG. 12 is a block diagram of a computing apparatus according to Embodiment 4 of the present disclosure;
Fig. 13 is a flow chart of a control method for a surgical robot according to Embodiment 5 of the present disclosure.

Reference signs in the drawings are listed as follows:
1-master console; 2-slave device; 3-memory; 4-processor; 21-mechanical arm; 22- cannula; 10-rotating component; 10A-first rotating component; 10S-coupling end; 20-axial biasing component; 30-first detection component; 40-controller; 50-drive input interface; 51-first input disk; 52-second input disk; 60-second detection component; 100-power apparatus; 100a-first housing; 200-surgical instrument; 210-long shaft; 211-end effector; 300a-second housing; 301-indication device; 302-axial vibration member; 600-axial movement gap; 3001-upper housing; 3002-lower housing; 3011-accommodation cavity; a-first angle threshold; b-second angle threshold; P1-first signal terminal; P2-second signal terminal; P3-third signal terminal; T-target portion.

### DETAILED DESCRIPTION

The present disclosure will be described clearly and thoroughly herein by accompanying with appended figures of some embodiments. Apparently, the embodiments are only component of the present disclosure, and are not the whole disclosure. For the person of ordinary skill in the art, other embodiments may be obtained based on the provided embodiments without any creative work, and the other embodiments are also covered by the present disclosure. The preferred embodiments of the present disclosure are illustrated in the accompanying drawings. However, it should be understood that the present disclosure can be implemented in many different forms and is not limited to the embodiments described herein. On the contrary, these embodiments are provided to enable a more thorough and comprehensive understanding of the present disclosure.

It should be noted that when an element is referred to as being "provided on" another element, it can be directly on the other element or intervening elements may also be present. When an element is considered to be "connected" to another element, it can be directly connected to the other element, or intervening elements may also be present. Similarly, when an element is considered to be "coupled" to another element, it can be directly coupled with the other element, or intervening elements may also be present. The terms "distal" and "proximal" are used herein as directional terms commonly used in the field of interventional medical devices, wherein "distal" refers to the end away from the operator during a surgical procedure, and "proximal" refers to the end closer to the operator during the surgical procedure.

It is important to understand that the terms "length," "width," "upper," "lower," "front," "rear," "left," "right," "vertical," "horizontal," "top," "bottom," "inner," "outer," etc., indicate orientation or positional relationships based on the orientation or positional relationships shown in the accompanying drawings. These terms are used solely for the convenience of describing and simplifying the description of the invention, and do not indicate or imply that the device or element referred to must have a specific orientation, be constructed and operated in a specific orientation. Therefore, they should not be construed as limitations on the invention.

Furthermore, the terms "first" and "second" are used only for descriptive purposes and should not be construed as indicating or implying relative importance or implicitly specifying the number of technical features indicated. Thus, features defined as "first" or "second" may explicitly or implicitly include one or more such features. In the description of the invention, the meaning of "multiple" refers to two or more, unless otherwise specifically and explicitly limited.

Unless otherwise defined, all technical and scientific terms used herein have the same meaning as commonly understood by one of ordinary skill in the art to which present disclosure belongs. The terminology used in the specification of the present disclosure is for the purpose of describing specific embodiments only and is not intended to limit the present disclosure. The term "and/or" as used herein includes any and all combinations of one or more of the associated listed items.

Referring to FIG.s 1 and 2, a surgical robot comprises a master console 1 and a slave device 2. The master console 1 comprises an operating part 1a through which the user controls the movement of the slave device 200. The master console 1 sends control commands to the slave device 2 to control it, and displays images acquired from the slave device 2. The slave device 2 responds to control commands sent by the master console 1 and performs corresponding operations. The slave device 2 can also be used to acquire images from inside the body.

The slave device 2 may include a mechanical arm 21, a power apparatus 100 disposed on the mechanical arm 21, a surgical instrument 200 disposed on the power apparatus 100, and a cannula 22 sheathing the long shaft 210 of the surgical instrument 200. The mechanical arm 21 is used to adjust the position of the surgical instrument 200, and the power apparatus 100 is used to drive the surgical instrument 200 to perform corresponding operations. It should be understood that the embodiments of the present disclosure are not limited to master-slave surgical robots, and the surgical robot may also adopt a construction that does not distinguish between master and slave devices and integrates operational functions into the slave device.

The surgical instrument 200 mainly comprises a long shaft 210, an end effector 211, and an instrument drive unit 212. The end effector 211 of the surgical instrument 200 is used to be inserted into the body and perform surgical operations and/or acquire images from inside the body through its distal end effector. The long shaft 210 of the surgical instrument 200 is inserted into the cannula 22, with its end effector 211 extending out of the cannula 22 and being driven by the power apparatus 100 to perform operations. The region of the long shaft 210 of the surgical instrument 200 located within the cannula 22 can be set as a rigid or flexible region according to requirements, and the cannula 22 can also be omitted.

The slave device 2 may include one or more mechanical arms 21each provided with one or more power apparatus 100, and one or more surgical instruments 200 can be mounted to each power apparatus 100.

On a mechanical arm 21 with only one surgical instrument 200, one power apparatus 100 can be connected to one surgical instrument 200, providing the driving force for the surgical instrument 200 to perform related operations. On a mechanical arm 21 with multiple surgical instruments 200, one power apparatus 100 can simultaneously be connected to multiple different surgical instruments 200, providing driving force for the multiple surgical instruments 200.

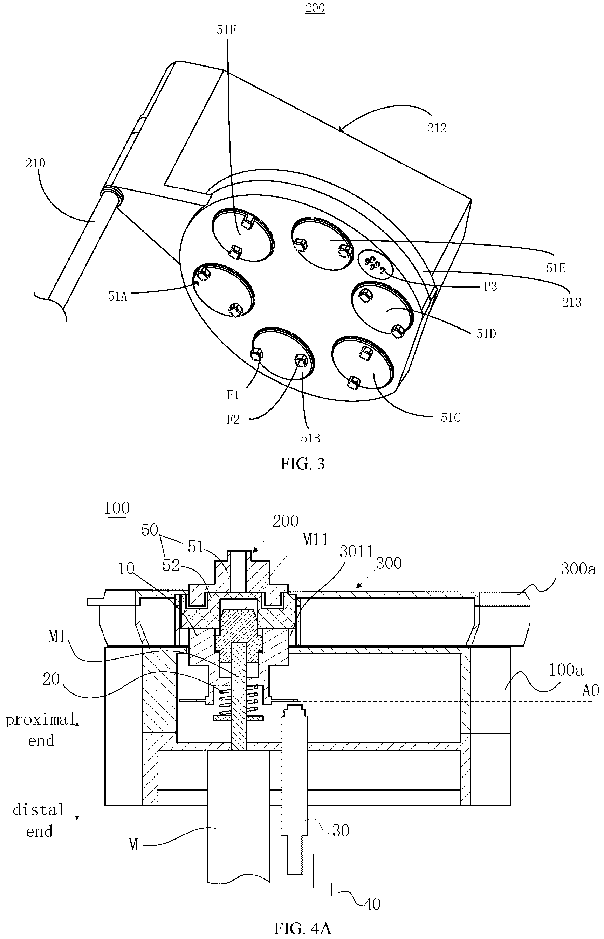

Referring to FIG. 3, to implement various surgical operations of the surgical instrument 200, each surgical instrument 200 comprises multiple first input disks 51, such as 51A-51F. Each power apparatus 100 engaged with the surgical instrument 200 further comprises multiple rotating components 10 driven by motors to rotate. When the surgical instrument 200 is drivingly coupled with the corresponding power apparatus 100, first input disks 51 are drivingly coupled with rotating components 10 respectively, thereby implementing torque transmission. Depending on the type of the end effector 211, the power apparatus 100 drives the end effector 211 to perform related surgical operations. FIG. 3 illustrates a case where the surgical instrument 200 comprises six first input disks 51A-51F. In other embodiments, the number of first input disks may vary. Surgical operations may include controlling the distal end of the long shaft 210 to perform deflection, rotation, pitch, and other operations. The end effector 211 may be surgical forceps, cautery devices, cutting devices, imaging devices, etc.

The first input disks 51 are disposed within the instrument housing 213, with the proximal end of the first input disks 51 rotatably connected to the instrument drive unit 212, and the distal end of the first input disks 51 exposed outside the instrument housing 213 and configured to receive driving input from the power apparatus 100, thereby driving the end effector 211 to move. Each first input disk 51 moves independently from other first input disks.

The following embodiment is described taking an example where the distal end of the instrument housing 213 of the surgical instrument 200 is mounted to the proximal end of the power apparatus 100. Under this circumstance, an instrument channel for the long shaft 210 at the distal end of the surgical instrument 200 to pass through is formed on the power apparatus 100. The first input disks 51 of the surgical instrument 200 are located at the distal end of the instrument housing 213, facing the end effector 211, while the rotating components 10 of the power apparatus 100 are located at the proximal end of the power apparatus 100. It should be understood that the present disclosure is also applicable to a case where the proximal end of the instrument housing 213 of the surgical instrument 200 is mounted to the distal end of the power apparatus 100. Under this circumstance, the first input disks 51 of the surgical instrument 200 are located at the proximal end of the instrument housing 213, facing away from the end effector 211, while the rotating components 10 of the power apparatus 100 are located at the distal end of the power apparatus 100.

### Embodiment 1

### An axial biasing component 20

To detect the drive output of the power apparatus 100 and provide stable and reliable drive input to the surgical instrument 200, in conjunction with FIGS. 4A to 5A, the present embodiment provides a power apparatus 100 that mainly comprises a first housing 100a, a rotating component 10, an axial biasing component 20, a first detection component 30, and a controller 40. The first housing 100a is used to fix the motor M, and each motor M is drivingly connected to a first input disk 51 and configured to provide a rotational power source for the first input disk 51. The rotating component 10 is rotatably disposed in the first housing 100a and comprises an axial coupling end 10S exposed outside the first housing 100a for being engaged with a drive-input interface 50. The drive-input interface 50, serving as the power input interface of the surgical instrument 200, can be part of the surgical instrument 200, a component independent from the surgical instrument 200, or part of the power apparatus 100.

The axial biasing component 20 is configured to provide an elastic bias toward the surgical instrument 200 for the rotating component 10, so that when the drive-input interface 50 is engaged with the rotating component 10, elastic contact can be maintained to achieve stable axial contact. The axial biasing component 20 experiences further deformation when the drive-input interface 50 is coupled with the rotating component 10, resulting in increased deformation. In the present embodiment, the axial biasing component 20 adopts a compression spring located on the side of the rotating component 10 further from the surgical instrument 200 (the distal end) than the rotating component 10, fixed to the rotation axis M1 of the motor M, and rotated with the rotation of the rotating component 10. When the rotating component 10 is not pressed, the compression of the axial biasing component 20 is minimal; when the rotating component 10 is pressed down, the compression of the axial biasing component 20 increases.

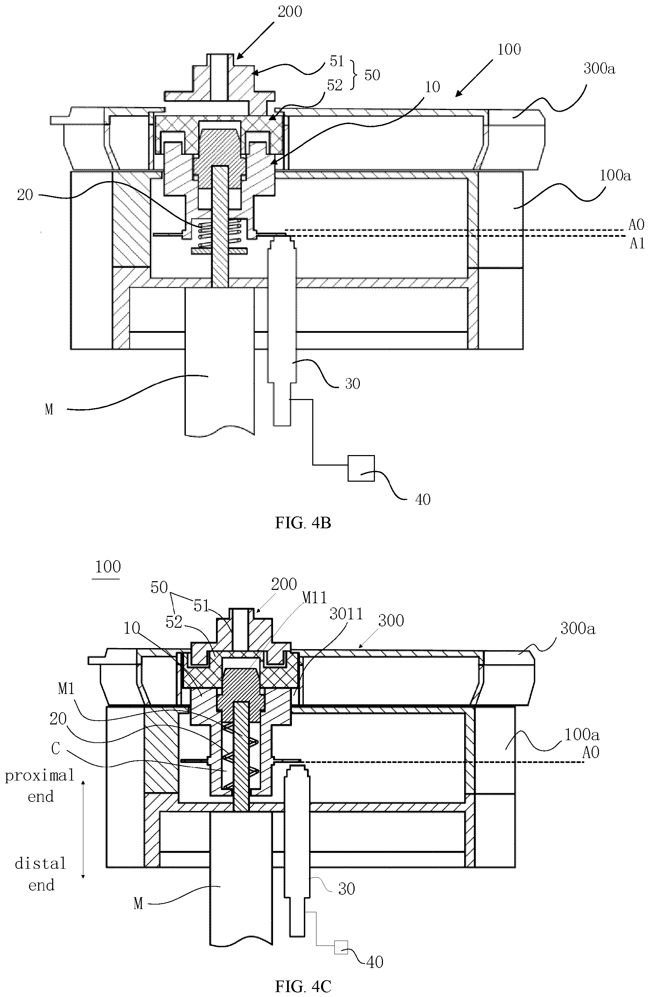

It is understood that in other embodiments, the axial biasing component 20 can also adopt other elastic component capable of deformation, such as rubber. For another example, referring to FIG. 4C, the axial biasing component 20 can also be disposed on the side of the rotating component 10 closer to the surgical instrument 200 (the proximal end) than the rotating component 10 and adopt a tension spring. One end of the axial biasing component 20 is fixed to the rotation axis M1 of the motor M, and the other end is fixed with the rotating component 10, pulling the rotating component 10 toward the surgical instrument 200. When the rotating component 10 is not pressed, the extension of the axial biasing component 20 is minimal; when the rotating component 10 is pressed down, the extension of the axial biasing component 20 increases. To avoid the tension spring extending out of the rotating component 10 and affecting engagement, a groove C can be provided at the end of the rotating component 10 to house the tension spring.

### A first detection component 30

In the initial state, the drive-input interface 50 is not mounted to the rotating component 10. In the axial direction of the rotating component 10, there is a gap between the detection end of the first detection component 30 and the rotating component 10. The first detection component 30 is configured to detect the position of the rotating component 10 or a certain part of the rotating component 10 in the axial direction of the rotating component 10. The controller 40 is configured to determine that the drive-input interface 50 is not coupled with the coupling end 10S of the rotating component 10 when the detected portion of the rotating component 10 is located at a position further from the surgical instrument 200 than a preset position (such as the A0 position shown in FIG. 4A). The rotating component 10 being at the preset position A0 is the critical state of coupling the drive-input interface 50 and the rotating component 10. Under this circumstance, the drive-input interface 50 compresses the axial biasing component 20 to provide torque for the surgical instrument 200. That is to say, the rotating component 10 is at the preset position A0 only when the drive-input interface 50 is coupled with the rotating component 10. Under other circumstances, the rotating component 10 is not at the preset position A0, and the drive-input interface 50 cannot be drivingly connected with the rotating component 10.

There are multiple ways for the first detection component 30 to detect the position of the rotating component 10 in the axial direction of the rotating component 10, which can comprise directly measuring the displacement (i.e., position) change of the rotating component 10 along the axial direction of the rotating component 10 or indirectly measuring the deformation of the axial biasing component 20.

In the present embodiment, the first detection component 30 detects the position change of the rotating component 10 along the axial direction of the rotating component 10. Specifically, there is a gap between the detection end of the first detection component 30 and the rotating component 10 along the axial direction of the rotating component 10. The detection end of the first detection component 30 is disposed on the side of the rotating component 10 further from the surgical instrument 200 than the rotating component 10, i.e., on the distal side of the rotating component 10. The first detection component 30 is a non-contact sensor configured to detect whether the detected portion of the rotating component 10 is sensed. For example, the first detection component 30 adopts a proximity sensor. Correspondingly, the controller 40 is configured to determine that the drive-input interface 50 is not coupled with the rotating component 10 when the first detection component 30 senses the detected portion of the rotating component 10.

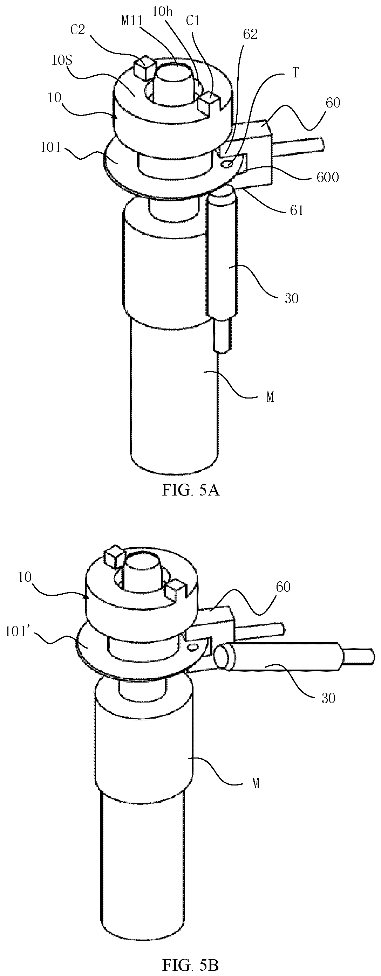

Referring to FIG. 4A, it illustrates a schematic diagram of the internal structure of the power apparatus in a coupled state of the surgical instrument. Referring to FIG. 4B, it illustrates a schematic diagram of the internal structure of the power apparatus in an uncoupled state of the surgical instrument according to Embodiment 1 of the present disclosure. FIG. 5A shows a schematic diagram of the main structure of a power apparatus. The drive-input interface 50 is coupled with the rotating component 10 through the engagement of concave and convex features facing each other. Referring to FIG. 5A, when there is no drive-input interface 50 above the rotating component 10, the axial biasing component 20 is in an initial deformation state, and the distance between the first detection component 30 and the rotating component 10 is relatively large, so that the first detection component 30 cannot detect the rotating component 10. Referring to FIG. 4B, when the drive-input interface 50 is mounted to the rotating component 10, the deformation of the axial biasing component 20 increases. When the concave and convex features for coupling on the drive-input interface 50 and the rotating component 10 are not aligned, the drive-input interface 50 presses the rotating component 10 towards the distal side, causing the axial biasing component 20 to reach its maximum deformation state. This reduces the distance between the first detection component 30 and the rotating component 10, and the actual position A1 of the rotating component 10 along the axial direction of the rotating component 10 has deviated towards the distal side from the preset position A0, thus being detected by the first detection component 30. Under this circumstance, the controller 40 determines that the drive-input interface 50 is not coupled with the rotating component 10. Referring to FIG. 4A, when the drive-input interface 50 is mounted to the rotating component 10 and the concave and convex features for coupling on the drive-input interface 50 and the rotating component 10 are aligned and engaged, the rotating component 10 rebounds towards the proximal side by a certain distance, which increases the distance between the first detection component 30 and the rotating component 10. The actual position of the rotating component 10 along the axial direction of the rotating component 10 reaches the preset position A0 and cannot be detected by the first detection component 30 anymore. Under this circumstance, the controller 40 determines that the drive-input interface 50 is coupled with the rotating component 10.

In FIG. 5A, the rotating component 10 has an annular detected portion 101. The first detection component 30 is configured to detect whether the detected portion 101 is sensed. The controller 40 is configured to determine that the drive-input interface 50 is not coupled with the rotating component 10 when the detected portion 101 is sensed. FIG. 5A shows a scenario where the detection end of the first detection component 30 is directly facing the distal surface of the rotating component 10. The first detection component 30 is located on one side of the rotating component 10 in the axial direction of the rotating component 10. When the rotating component 10 is pressed to move in a direction that increases the deformation of the axial biasing component 20 (i.e., in a direction opposite to the surgical instrument 200), the distance between the detected portion 101 and the detection end of the first detection component 30 gradually decreases. It can be understood that the position of the first detection component 30 can be changed. For example, FIG. 5B shows another power apparatus. In FIG. 5B, the rotating component 10 has an annular detected portion 101'. The first detection component 30 is disposed on the outer side of the rotating component 10 in the radial direction and faces the central axis of the rotating component 10 to detect the annular outer circumferential surface of the detected portion 101'. The annular outer circumferential surface has a larger radial dimension than the adjacent outer circumferential surface of the rotating component 10. When the rotating component 10 is pressed to move in a direction that increases the deformation of the axial biasing component 20, the detected portion 101' of the rotating component 10 can move axially from a distant position towards the detection end of the first detection component 30. The distance between the detected portion 101' and the detection end of the first detection component 30 gradually decreases. When the detected portion 101' approaches the first detection component 30, it can be detected by the first detection component 30. During the subsequent continued pressing of the rotating component 10, the detected portion 101' will gradually move away from the detection end of the first detection component 30, and the distance between the detected portion 101' and the detection end of the first detection component 30 will gradually increase. The position of the first detection component 30 can be set such that when it detects the detected portion 101' directly opposite to it, the rotating component 10 reaches the preset position A0. Correspondingly, the controller 40 is configured to determine that the drive-input interface 50 is coupled with the rotating component 10 when the detected portion 101' is detected. The detected portion 101' can be the same as the detected portion 101 or can be a different portion from the detected portion 101.

It can be understood that in other embodiments, the first detection component 30 may not adopt a proximity sensor instead of a distance sensor. The first detection component 30 is configured to detect the distance between it and the detected portion 101. The controller 40 is configured to determine that the drive-input interface 50 is not coupled with the rotating component 10 when the first detection component 30 detects that the distance between it and the rotating component 10 is less than a preset distance; determine that the drive-input interface 50 is coupled with the rotating component 10 when the first detection component 30 detects that the distance between it and the rotating component 10 is equal to the preset distance; and determine that the drive-input interface 50 is not coupled with the rotating component 10 when the first detection component 30 detects that the distance between it and the rotating component 10 is greater than the preset distance. The proximity sensor includes but is not limited to capacitive, inductive, and photoelectric sensors, while the distance sensor includes but is not limited to optical sensors, infrared sensors, and ultrasonic sensors.

### Adaptor assembly 300

To provide a suitable sterile environment during surgical procedure of the surgical robot, it is necessary to establish an intermediate barrier between sterile and non-sterile instruments. Typically, the mechanical arm 21 and power apparatus 100 of the operating equipment 2 are non-sterile, while the surgical instrument 200 needs to be sterile. In the present embodiment, an adaptor assembly 300 is provided between the non-sterile power apparatus 100 and the sterile surgical instrument 200. Specifically, the adaptor assembly 300 can serve as part of a sterile barrier isolating the surgical instrument from other parts of the equipment during usage. The sterile barrier separates the non-sterile power apparatus 100 and the sterile surgical instrument 200, while the power (such as motor torque output) from the power apparatus 100 is transmitted through the adaptor assembly 300 to the surgical instrument 200, which enables the surgical instrument 200 to perform corresponding surgical operations while ensuring sterile isolation.

The surgical instrument 200 is provided with at least one first input disk 51, the adaptor assembly 300 is provided with at least one second input disk 52, and the power apparatus 100 is provided with at least one rotating component 10. The number of rotating components 10, the number of first input disks 51 on the surgical instrument 200, and the number of second input disks 52 on the adaptor assembly 300 are equal, allowing the first input disks 51 to be coupled with the rotating components 10 through the second input disks 52 respectively.

Referring to FIG.s 6A to 7, in the present embodiment, the adaptor assembly 300 specifically includes a second housing 300a detachably mounted the a first housing 100a and multiple second input disks 52, such as 52A-52F, rotatably disposed on the second housing 300a. In the present embodiment, the first input disk 51 and the second input disk 52 form a drive-input interface 50. The second input disk 52 serves only as part of the drive-input interface 50. The coupling of the first input disk 51, the second input disk 52, and the rotating component 10 in pairs is referred to as the coupling of the drive-input interface 50 with the rotating component 10. If only the second input disk 52 is coupled with the rotating component 10, and the first input disk 51 is not coupled with the second input disk 52, the drive-input interface 50 is still not coupled with the rotating component 10. In this case, the first detection component 3 can still detect the rotating component 10.

As a preferred implementation, the second input disk 52 and the rotating component 10 are coupled through the engagement of concave and convex features on end surfaces facing each other. The first input disk 51 on the surgical instrument 200 and the second input disk 52 are coupled through the engagement of concave and convex features on end surfaces facing each other to transmit torque between the rotating component 10 and the surgical instrument 200. The second input disk 52 has a degree of freedom along the axial direction of the second housing 300a, that is, the second input disk 52 can be rotated relative to the second housing 300a or move along the axial direction of the second housing 300a, which allows the second input disk 52 to be elastically contacted and axially displaced by the axial biasing component 20, providing two contact states: coupled and not coupled with the rotating component 10.

Correspondingly, the controller 40 is configured to determine whether the second input disk 52 is coupled with the rotating component 10 based on the detection result from the first detection component 30 after the second housing 300a is mounted to the power apparatus. When the second input disk 52 is coupled with the rotating component 10, and the surgical instrument 200 is mounted to the power apparatus, the controller 40 determines whether the first input disk 51 is coupled with the second input disk 52 based on the detection result from the first detection component 30. This allows the detection of the coupling states of both the second input disk 52 on the adaptor assembly 300 and the first input disk 51 on the surgical instrument 200 using only one first detection component 30 for each rotating component 10.

To achieve axial coupling, the coupling end 10S of the rotating component 10 is provided with first coupling features C1, C2, the lower surface (distal end) of the second input disk 52 is provided with second coupling features D1, D2, the upper surface (proximal end) of the second input disk 52 is provided with third coupling features E1, E2, and the lower surface (distal end) of the first input disk 51 is provided with fourth coupling features F 1, F2. The first coupling features C1, C2 are convex or concave features and the second coupling features D1, D2 are concave or convex features adaptive to be engaged with the first coupling features C1, C2 correspondingly. The third coupling features E1, E2 are convex or concave features and the fourth coupling features F1, F2 are concave or convex features adaptive to be engaged with the third coupling features E1, E2. After the first coupling features C1, C2 are engaged with the second coupling features D1, D2, and the third coupling features E1, E2 are engaged with the fourth coupling features F 1, F2, the torque of the rotating component 10 can be transmitted to the first input disk 51.

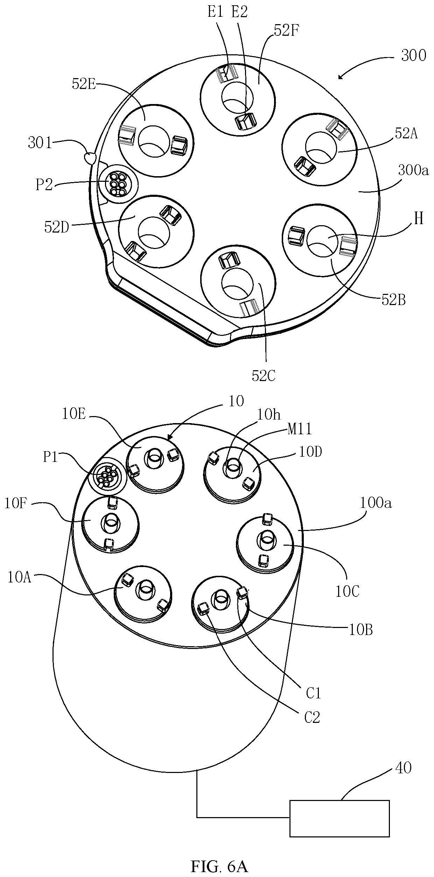

FIG. 6A illustrates a structural schematic diagram of the power apparatus 100 and its adapter assembly 300. The illustrated power apparatus 100 includes six rotating components 10, namely 10A-10F. Multiple rotating components 10 are mounted in the first housing 100a controlled and actuated by controllers 40 respectively. Each controller 40 independently controls and drives a surgical instrument 200, for example, rotating components 10 controls operations such as rotation, deflection, pitch, and end effector opening/closing of the surgical instrument 200 respectively. In other embodiments, the number of controllers 40 can also be varied as needed, for example, one controller 40 may control the rotation of all rotating components 10 in the power apparatus 100.

Referring to FIG.s 6A to 7, the second housing 300a of the adapter assembly 300 includes an upper housing 3001 disposed at its proximal end and a lower housing 3002 disposed at its distal end. The upper housing 3001 and the lower housing 3002 cooperate to form multiple accommodation cavities 3011 each designed for accommodating one second input disk 52. The upper housing 3001 comprises a first edge portion 3010 that restricts the axial movement of the second input disk 52 towards the distal end, while the lower housing 3002 comprises a second edge portion 3020 that restricts the axial movement of the second input disk 52 towards the proximal end. The axial dimension of the second input disk 52 is less than the axial dimension of the accommodation cavity 3011, allowing the second input disk 52 to move axially within the accommodation cavity 3011.

Referring to FIG.s 5A, 6A, and 6B, a free end of the rotation axis M1 of the motor M extends out of the rotating component 10, protruding further from the rotating component 10 than the coupling end 10S. An axis hole H is provided at the axial center of the second input disk 52 of the adapter assembly 300. When the adapter assembly 300 is mounted to the first housing 100a, the free end of the rotation axis M1 is inserted into the axis hole H, which implements the pre-alignment of the mounting of the adapter assembly 300 and facilitates the radial alignment between the rotating component 10 and the second input disk 52. The axis hole H can be a groove located on the surface of the distal end of the second input disk 52. Specifically, the free end of the rotation axis M1 is fixed with an axis cap M11 and the center of the rotating component 10 is provided with a central hole 10h for the rotation axis M1 to pass through. The axis cap M11 is fixed to the free end of the rotation axis M1 and at least partially accommodated within the central hole 10h providing radial restraint to the rotating component 10. Under the action of the axial biasing component 20, the rotating component 10 elastically contacts the axis cap M11 in the axial direction. The axis cap M11 allows the rotating component 10 to move axially but prevents it from rotating relative to the rotation axis M1.

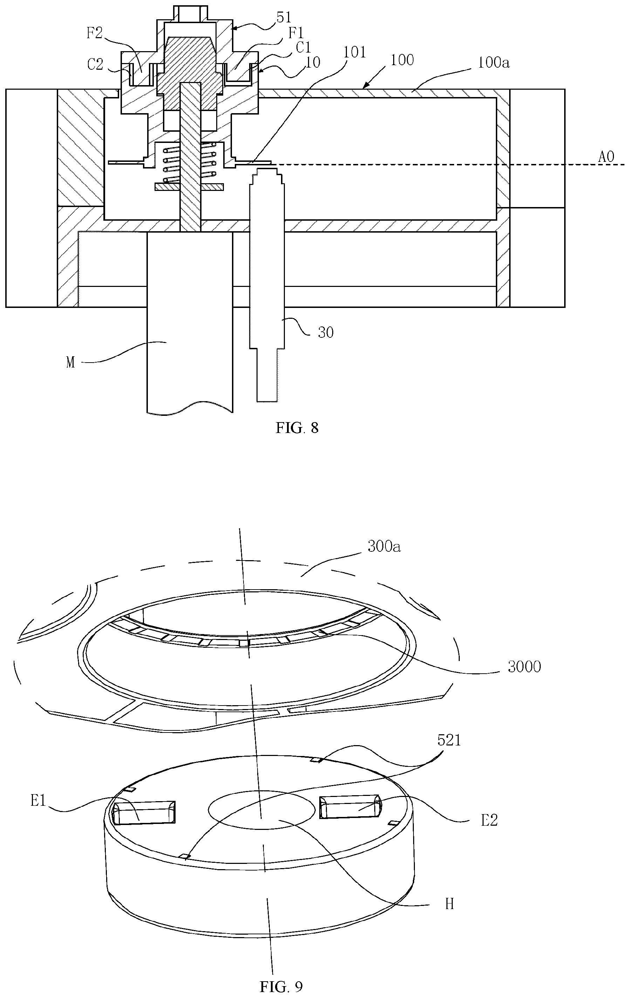

It is understood that the adapter assembly 300 is not mandatory. In some embodiments, the adapter assembly 300 can be omitted. Referring to FIG. 8, the first input disk 51 is directly engaged and coupled with the rotating component 10. Under this circumstance, the drive-input interface 50 includes a first input disk 51 disposed on the surgical instrument 200 and coupled with the rotating component 10 through the engagement of concave and convex features (C1, C2, and F1, F2) on end surfaces facing each other. Correspondingly, the controller 40 is configured to determine whether the first input disk 51 is coupled with the rotating component 10 based on the detection result from the first detection component 30 after the surgical instrument 200 is mounted to the power apparatus. This detection process is consistent with the previous process, i.e., when the detected portion of the rotating component 10 is located at a position further from the surgical instrument 200 than the preset position A0, it is determined that the drive-input interface 50 is not coupled with the coupling end 10S of the rotating component 10.

### Signal Terminals P1, P2, P3

To determine the coupling state of the drive-input interface 50 and the rotating component 10, the power apparatus can include various signal terminals. Referring to FIG. 6A, the first housing 100a of the power apparatus 100 is provided with a first signal terminal P1, which can be conducted by contacting with a signal terminal on the drive-input interface 50. The controller 40 is configured to determine that the drive-input interface 50 is mounted to the power apparatus based on the signal indicating conduction between the first signal terminal P1 and the signal terminal of the drive-input interface 50.

In the power apparatus 100 with the adapter assembly 300, the adapter assembly 300 is configured to transmit torque between the rotating component 10 and the surgical instrument 200. The adapter assembly 300 includes a second signal terminal P2 that passes through both the proximal and distal surfaces of the second housing 300a. When the adapter assembly 300 is mounted to the first housing 100a of the power apparatus 100, the second signal terminal P2 can be conducted by contacting with the first signal terminal P1 on the first housing 100a. Subsequently, when the surgical instrument 200 is mounted to the adapter assembly 300, a third signal terminal P3 on the surgical instrument 200 can be conducted by contacting with the second signal terminal P2, thereby forming a pathway connected to the first signal terminal P1. The controller 40 is configured to determine that the adapter assembly 300 is mounted to the first housing 100a based on the signal indicating conduction between the second signal terminal P2 and the first signal terminal P1. After the adapter assembly 300 is mounted to the first housing 100a of the power apparatus 100, the controller 40 is configured to determine that the surgical instrument 200 is mounted to the adapter assembly 300 based on the signal indicating conduction between the third signal terminal P3 of the surgical instrument 200 and the first signal terminal P1.

In the power apparatus 100 without the adapter assembly 300, when the surgical instrument 200 is mounted to the first housing 100a of the power apparatus 100, its third signal terminal P3 can be conducted by contacting with the first signal terminal P1. The controller 40 is configured to determine that the surgical instrument 200 is mounted to the power apparatus 100 based on the signal indicating conduction between the third signal terminal P3 of the surgical instrument 200 and the first signal terminal P1.

### Indication device 301

Referring to FIG.s 6A and 7, although the controller 40 can be configured to determine the mounting of the adapter assembly 300/surgical instrument 200 based on the conduction states of various signal terminals, to further facilitate the operator visualization of the mounting state of the adapter assembly 300 and the surgical instrument 200, the power apparatus 100 in the present embodiment further comprises an indication device 301, which can be disposed on the outer wall of the second housing 300a for easy observation. Since the first housing 100a is provided with a first signal terminal P1, the adapter assembly 300 is provided with a second signal terminal P2, and the surgical instrument 200 is provided with s a third signal terminal P3, the indication device 301 is disposed on the adapter assembly 300 and electrically connected to the second signal terminal P2. The indication device 301 can be conducted with the detection circuit comprising the first signal terminal through the second signal terminal P2, thereby emitting indication based on the coupling state of the second input disk 52 of the adapter assembly 300 and the first input disk 51 of the surgical instrument 200. The indication device 301 can be an indicator light or a loudspeaker, etc. The second signal terminal P2 can be conducted with the first signal terminal P1 after the adapter assembly 300 is mounted to the first housing 100a, and can be conducted with the third signal terminal P3 after the surgical instrument 200 is mounted to the adapter assembly 300. The controller 40 is configured to switch the indication state of the indication device based on the detection result from the first detection component 30 after the adapter assembly 300 is mounted to the first housing 100a and/or after the surgical instrument 200 is mounted to the adapter assembly 300. For example, when it is detected that the second input disk 52 of the adapter assembly 300 is coupled with the rotating component 10, the indication device 301 turns from on to off. When the first input disk 51 of the surgical instrument 200 is coupled with the second input disk 52, the indication device 301 also turns from on to off.

### A second detection component 60

Referring to FIG. 5A, to accurately detect the zero position (i.e., the initial position) of the rotating component 10 and facilitate precise control of the rotation angle of the motor M, the power apparatus 100 is provided with a second detection component 60, and a target portion T is disposed on the rotating component 10 correspondingly. When the motor M drives the rotating component 10 to be rotated to the zero position, the target portion T is rotated to be directly opposite to the second detection component 60, thereby determining that the rotating component 10 has returned to the zero position. Based on the zero position, the rotation angle of the rotating component 10 can be determined, which facilitates the subsequent mounting and coupling of the surgical instrument 200. The controller 40 is configured to determine that the rotating component 10 is rotated to the zero position when the target portion T is rotated with the rotating component 10 to be detected by the second detection component 60.

The target portion T can be a through-hole in the axial direction of the rotating component 10. The second detection component 60 includes a transmitting end 61 and a receiving end 62 disposed on two sides of the target portion T along the axial direction of the rotating component 10. When the signal emitted by the transmitting end 61 is received by the receiving end 62, it is indicated that the target portion T is rotated to be between the transmitting end 61 and the receiving end 62; otherwise, it is indicated that the target portion T is not directly opposite to the second detection component 60. The target portion T can be disposed on the annular detected portion 101, and the transmitting end 61 and the receiving end 62 are located on two sides of the detected portion 101 along the axial direction of the rotating component 10, clamping the detected portion 101 therebetween. An axial movement gap 600 reserved for the detected portion 101 is formed between the transmitting end 61 and the receiving end 62.

In other embodiments, the target portion T can be another traceable identifier. For example, the target portion T can be a magnet, and the second detection component 60 can be a Hall sensor. By utilizing the Hall sensor to detect changes in the magnetic field generated by the magnet nearby, the rotation angle of the rotating component 10 can be identified, thereby determining the zero position.

Typically, when the power apparatus 100 is powered on, the motor M drives the rotating component 10 to be rotated to the zero position. During this process, the position of the zero position and whether the motor M is working properly can be determined by an encoder of the motor etc.. At the same time, it can also avoid the impact of motor M failure on the accuracy of detecting the mounting state of the subsequent adapter assembly 300 and surgical instrument 200. The controller 40 is configured to control the rotation of the rotating component 10 until the target portion T is detected by the second detection component 60, so that the rotating component 10 stops at the zero position, after which the drive-input interface 50 is mounted. In some embodiments, the rotating component 10 does not return to the zero position when the power apparatus 100 is powered on.

In the power apparatus 100 with the adapter assembly 300, the adapter assembly 300 is detachably mounted to the first housing 100a. After the adapter assembly 300 is mounted to be drivingly coupled with the rotating component 10, the motor M can also be controlled to drive the rotating component 10 to be rotated to the zero position. After the end effector 211 of the surgical instrument 200 passes through the cannula 22, various joints of the surgical instrument 200 are proximately straightened. When the adapter assembly 300 is driven to be engaged with the rotating component 10 and each rotating component 10 is at the zero position, the position of the concave and convex features of the second input disk 52 of the adapter assembly 300 and the first input disk 51 of the straightened surgical instrument 200 correspond exactly. Therefore, returning the rotating component 10 to the zero position before mounting the surgical instrument 200 allows the surgical instrument 200 to be directly drivingly engaged with the adapter assembly 300 after being mounted to the adapter assembly 300, or only requires a small adjustment of the rotation angle of the rotating component 10 to be engaged with the corresponding first input disk 51, which can improve the engagement efficiency of the surgical instrument. The controller 40 is configured to control the rotating component 10 to be rotated towards the zero position when the adapter assembly 300 is mounted to the first housing 100a and the first detection component 30 detects that the second input disk 52 of the adapter assembly 300 is drivingly engaged with the rotating component 10, allowing the target portion T to be detected by the second detection component 60. In some embodiments, if the rotating component 10 does not return to the zero position before mounting the surgical instrument 200, it will be necessary to align and engage the first input disk 51 with the corresponding second input disk 52 after mounting the surgical instrument 200, which will take more time.

Referring to FIG. 6A, the rotating component 10 includes a first rotating component 10A and a second rotating component 10B. Each rotating component 10 corresponds to one drive-input interface 50, one first detection component 30, and one second detection component 60. The first rotating component 10A includes a rotating component 10 configured to drive the end effector of the surgical instrument 200 to perform non-self-rotation movements such as pitch, yaw, opening, and closing. The second rotating component 10B comprises a rotating component 10 configured to drive the end effector of the surgical instrument 200 to perform self-rotation movements. After the adapter assembly 300 is mounted to the first housing 100a and the second input disk 52 is drivingly coupled with the rotating component 10, the surgical instrument 200 can be mounted subsequently. After the surgical instrument 200 is mounted to and drivingly coupled with the adapter assembly 300, the first rotating component 10A can be rotated to the zero position. Unlike the first rotating component 10A, the second rotating component 10B can be rotated such that the target portion T is in a preset angular range α. The preset angular range α represents the master-slave operation limit range of the operation part 1a and the slave surgical instrument 200. If the target portion T is outside the preset angular range α, it will be impossible to align the posture of the operation part 1a with the posture of the slave surgical instrument 200 during the subsequent master-slave alignment before the operation, leading to the automatic exit of the master-slave alignment procedure and affecting the surgeon's operational experience. The subsequent master-slave alignment procedure can be performed only when the target portion T is in the preset angular range α.

Therefore, when the surgical instrument 200 is mounted to the adapter assembly 300 and the first detection component 30 detects that the first input disk 51 is coupled with the first rotating component 10A, if the target portion T is detected by the second detection component 60, the controller 40 can be configured to stop rotating the first rotating component 10A; otherwise, the controller 40 can be configured to control the first rotating component 10A to be rotated until the target portion T is detected by the second detection component 60. When the surgical instrument 200 is mounted to the adapter assembly 300 and the first detection component 30 detects that the first input disk 51 is coupled with the second rotating component 10B, if the target portion T is in the preset angular range α, the controller 40 can be configured to stop rotating the second rotating component 10B; otherwise, the controller 40 can be configured to control the second rotating component 10B to be rotated until the target portion T is in the preset angular range α. The preset angular range α can be defined based on data from the encoder of the motor M, and whether the target portion T is in the preset angular range α can be calculated based on the rotation angle of the motor M.

During the actual mounting process, after the adapter assembly 300 is mounted to the first housing 100a, or after the surgical instrument 200 is mounted to the adapter assembly 300, it is generally difficult to achieve one-time alignment between the first coupling features C1, C2 and the second coupling features D1, D2, as well as one-time alignment between the third coupling features E1, E2 and the fourth coupling features F1, F2. In the present embodiment, when the adapter assembly 300 is mounted to the first housing 100a and the second input disk 52 is not coupled with the rotating component 10, the controller 40 is configured to control the rotating component 10 to be rotated in a preset first manner. When the rotating component 10 is rotated to be coupled with the second input disk 52, the controller 40 is configured to interrupt the rotation of the rotating component 10 in the preset first manner. The preset first manner can comprise scanning by rotation in forward and reverse directions for M times. The rotation from the initial position to the extreme position in the forward direction and then returning to the initial position is referred to as scanning. One rotation from the initial position to the extreme position in the forward direction or one rotation from the extreme position to the initial position one time is referred to as a unidirectional scanning. The angle of unidirectional scanning of the rotating component 10 is equal to or less than a first angle threshold a. Subsequently, when the first input disk 51 is not coupled with the second input disk 52, the controller 40 is configured to control the rotating component 10 to be rotated in a preset second manner. When the rotating component 10 is rotated to be coupled with the first input disk 51, the controller 40 is configured to interrupt the rotation of the rotating component 10 in the preset second manner. The preset second manner can comprise scanning by rotation in forward and reverse directions for N times, wherein angle of unidirectional scanning of the rotating component 10 is equal to or less than a second angle threshold b. M and N are both equal to or greater than 1.

During the mounting process of the surgical instrument 200, the preset rotation of the rotating component 10 is interrupted immediately after being rotated to be coupled with the second input disk 52, preventing the instrument from being accidentally driven after being drivingly coupled with the power apparatus 100, avoiding potential harm to the human body. Moreover, during the mounting process of the adapter assembly 300, the preset rotation is interrupted immediately after the second input disk 52 is successfully coupled, eliminating unnecessary movements and improving mounting efficiency.

After the adapter assembly 300 is mounted, if the first coupling features C1, C2 are not aligned with the second coupling features D1, D2, the biasing force applied by the axial biasing component to the rotating component 10 will elastically press the second input disk 52 against the proximal inner surface of the second housing 300a. By controlling the rotating component 10 to be rotated repeatedly in forward and reverse directions, the rotating component 10 can be rotated relative to the second input disk 52, aligning the first coupling features C1, C2 with the second coupling features D1, D2. Similarly, after the surgical instrument 200 is mounted, if the third coupling features E1, E2 are not aligned with the fourth coupling features F 1, F2, by controlling the rotating component 10 to be rotated repeatedly in forward and reverse directions, the second input disk 52 can be rotated relative to the first input disk 51, aligning the third coupling features E1, E2 with the fourth coupling features F1, F2. Before the surgical instrument 200 is mounted, the rotating component 10 can drive the second input disk 52 of the adapter assembly 300 to be rotated freely. After the surgical instrument 200 is mounted, the surgical instrument 200 may also be rotated with the rotating component 10 without coupling. To avoid uncontrolled accidental movement of the end effector at the wound site, the second angle threshold b is less than the first angle threshold a.

It is understood that in some embodiments, the controller 40 may also be configured to control the rotating component 10 to be rotated in both forward and reverse directions for scanning regardless of whether the second input disk 52 is coupled with the rotating component 10 when the adapter assembly 300 is detected to be mounted to the first housing 100a. The unidirectional scanning angle of the rotating component 10 is equal to or less than a first angular threshold a. When the surgical instrument 200 is detected to be mounted to the adapter assembly 300, the controller 40 is configured to control the rotating component 10 to be rotated in both forward and reverse directions for scanning regardless of whether the first input disk 51 is coupled with the second input disk 52. The unidirectional scanning angle of the rotating component 10 is equal to or less than a second angular threshold b, which is less than the first angular threshold a.

Furthermore, in one embodiment, the first rotating component 10A needs to be rotated to a zero position after the surgical instrument 200 is mounted to and drivingly coupled with the adapter assembly 300. The controller 40 is configured to control the first rotating component 10A to be rotated in a preset third manner when the first rotating component 10A is coupled with its corresponding second input disk 52 and the corresponding first input disk 51 is mounted to the corresponding second input disk 52 but not coupled with the first rotating component 10A. During this process, once the first detection component 30 detects that the first input disk 51 is coupled with the second input disk 52, the controller 40 is configured to immediately interrupt the rotation of the first rotating component 10A in the preset third manner and control the rotating component 10 to be rotated towards the zero position. The controller 40 is further configured to stop the rotation of the first rotating component 10A when the target portion T of the first rotating component 10A is detected by the second detection component 60. When the second rotating component 10B is coupled with its corresponding second input disk 52 and the corresponding first input disk 51 is mounted to the corresponding second input disk 52 but not coupled with the second rotating component 10B, the controller 40 is configured to control the second rotating component 10B to be rotated in a preset fourth manner. When the second rotating component 10B is rotated to be coupled with the corresponding first input disk 51, the controller 40 is configured to interrupt the rotation of the second rotating component 10B in the preset fourth manner and control the second rotating component 10B to be rotated until the target portion T is in the preset angular range α. That is, once the first detection component 30 detects that the first input disk 51 is coupled with the second input disk 52, regardless of whether the rotating component 10 is about to be rotated forward or reverse, the rotating component 10 is directly rotated towards the zero position until the target portion T is detected by the second detection component 60. The orientation of the zero position can be determined in conjunction with an encoder when the power apparatus 100 is powered on. This allows the rotating component 10 to return to the zero position in the shortest time and rotation path after the first input disk 51 is coupled with the second input disk 52 without performing unnecessary movements or waiting for the rotating component 10 to finish scanning a certain angular threshold, thereby improving mounting efficiency and safety.

In some embodiments, when the surgical instrument 200 is mounted to the adapter assembly 300 and the first detection component 30 detects that the first input disk 51 is coupled with the first rotating component 10A, the first rotating component 10A immediately stops rotating and does not return to the zero position. The controller 40 is configured to control the first rotating component 10A to be rotated in a preset third manner when the first rotating component 10A is coupled with a second input disk 52 and the corresponding first input disk 51 is mounted to the corresponding second input disk 52 but not coupled with the first rotating component 10A. When the first rotating component 10A is rotated to be coupled with the corresponding first input disk 51, the controller 40 is configured to interrupt the rotation of the first rotating component 10A in the preset third manner and stop rotation of the first rotating component 10A without controlling the rotating component 10 to be rotated towards the zero position. When the second rotating component 10B is coupled with its corresponding second input disk 52 and the corresponding first input disk 51 is mounted to the corresponding second input disk 52 but not coupled with the second rotating component 10B, the controller 40 is configured to control the second rotating component 10B to be rotated in a preset fourth mode. When the second rotating component 10B is rotated to be coupled with the corresponding first input disk 51, the controller 40 is configured to interrupt the rotation of the second rotating component 10B in the preset fourth mode and control the target portion T of the second rotating component 10B to be rotated in a preset angular range α. That is, once the first detection component 30 detects that the first input disk 51 is coupled with the second input disk 52, regardless of whether the rotating component 10 is about to be rotated forward or reverse, the controller 40 is configured to immediately stop rotating and waits for the next master-slave alignment command. When all drive-input interfaces 50 of the surgical instrument 200 are coupled with the corresponding rotating components 10 of the power apparatus and the target portion T of the second rotating component 10B is in the preset angular range α, the next master-slave alignment action can be performed to align the posture of the operating part 1a with the posture of the slave device 2.

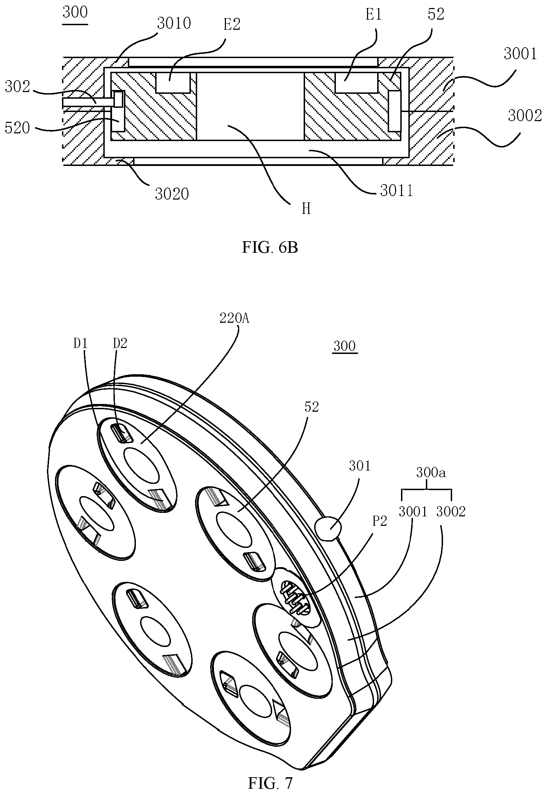

Referring to FIG. 6B, additionally, considering that after the adapter assembly 300 is mounted to the first housing 100a, even if the rotating component 10 is controlled to be rotated repeatedly in forward and reverse directions for M times, the second input disk 52 still cannot be coupled with the rotating component 10, or after the rotating component 10 is controlled to be rotated repeatedly in forward and reverse directions for N times, the first input disk 51 still cannot be coupled with the second input disk 52, the adapter assembly 300 of the present embodiment further includes an axial vibration component 302, which partially extends into the accommodation cavity 3011. When the second input disk 52 cannot be coupled with the rotating component 10, or the first input disk 51 cannot be coupled with the second input disk 52, the axial vibration component 302 can be controlled to vibrate the second input disk 52 along the axial direction of the adapter assembly 300 until the corresponding coupling features being engaged with each other. For example, the axial vibration component 302 can be a linear motor, fixed on the second housing 300a, and electrically connected to the controller 40 via the second signal terminal P2.

For example, an annular recess 520 can be formed on the outer circumferential surface of each second input disk 52. Part of the axial vibration component 302 is disposed in the second housing 300a, while another part extends into the recess 520. When the second input disk 52 is coupled with the rotating component 10 and the first input disk 51, the rotating component 10 is at a preset position A0, and the axial vibration component 302 may not contact the recess 520, thus not affecting the rotation of the second input disk 52. When the rotating component 10 is not at the preset position A0, the axial vibration component 302 drives the second input disk 52 to vibrate slightly in the axial direction and cooperates with the forward and reverse rotations of the rotating component 10, so that the rotating component 10, the second input disk 52, and the first input disk 51 can be coupled. Therefore, when the adapter assembly 300 is mounted and the first coupling features C1 and C2 are not aligned with the second coupling features D1 and D2, the controller 40 can be configured to control the rotating component 10 to be rotated repeatedly in forward and reverse directions and activate the axial vibration component 302 to axially vibrate the rotating component 10 relative to the second input disk 52, so as to align the first coupling features C1 and C2 with the second coupling features D1 and D2. Similarly, when the surgical instrument 200 is mounted and the third coupling features E1 and E2 are not aligned with the fourth coupling features F1 and F2, the controller 40 is configured to control the rotating component 10 to be rotated repeatedly in both forward and reverse directions and activate the axial vibration component 302, so that the second input disk 52 is rotated and axially vibrated relative to the first input disk 51, aligning the third coupling features E1 and E2 with the fourth coupling features F1 and F2.

Referring to FIG. 9, another adapter assembly 300 of the present embodiment is illustrated. Considering that in some extreme cases, after the adapter assembly 300 is mounted to the first housing 100a, even if the rotating component 10 is rotated in a preset manner, the rotating component 10 remains not coupled with the second input disk 52, and their concave and convex features are not engaged and remain in a misaligned state. The rotating component 10, under the action of the axial biasing component 20, drives the uncoupled second input disk 52 to be rotated against the reverse friction force from the second housing 300a of the adapter assembly 300, which may mislead people into thinking that the rotating component 10 is coupled with the second input disk 52. To eliminate this false coupling state, in the present embodiment, a plurality of second protrusions 3000 are provided on the surface of the second housing 300a facing the surface where the third coupling features E1 and E2 are located (i.e., the first edge portion 3010), and these second protrusions 3000 are spaced apart along the circumference of each accommodation cavity 3011. A plurality of first protrusions 521 are provided on the surface of the second input disk 52 where the third coupling features E1 and E2 are located. These first protrusions 521 are disposed at the edge of the second input disk 52 and do not contact the fourth coupling features F1 and F2. That is, the radius of the circle where the first protrusions 521 are located is greater than the larger one of the radius of the circles where the fourth coupling features F1 and F2 are located, so that the first protrusions 521 only restrict the relative rotation between the second input disk 52 and the second housing 300a without interfering with the first input disk 51 of the surgical instrument 200.

After the adapter assembly 300 is mounted to the first housing 100a, the second input disk 52 is pressed against the inner surface of the second housing 300a (i.e., the inner surface of the upper housing 3001) by the rotating component 10 under the biasing action of the axial biasing component 20. The first protrusions 521 of the second input disk 52 are inserted into the gaps between the second protrusions 3000 of the second housing 300a. When the rotating component 10 drives the second input disk 52 to be rotated in an uncoupled state, the first protrusions 521 are rotated within the gaps between the second protrusions 3000 until they are blocked by the second protrusions 3000. Even if one second protrusion 3000 cannot exert sufficient resistance to stop the second input disk 52 temporarily, the second input disk 52 can be decelerated after the first protrusions 521 pass through multiple second protrusions 3000, so that the rotating component 10 and the second input disk 52 can be truly coupled. Once the rotating component 10 and the second input disk 52 are coupled, the resistance between the first protrusions 521 can be overcome and the second input disk 52 can be rotated along the rotating component 10 and the second input disk 52.

### Embodiment 2

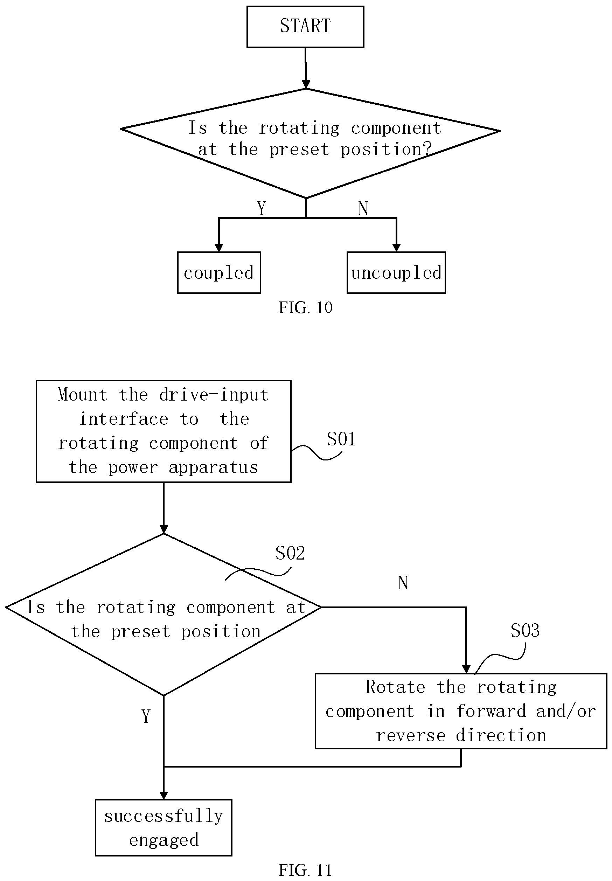

Referring to FIG. 10, the present embodiment provides a detecting method for the engagement state of a power apparatus of a surgical robot. The power apparatus 100 includes a first housing 100a, a rotating component 10 rotatably disposed on the first housing 100a and comprising a coupling end 10S in the axial direction, an axial biasing component 20 configured to provide an elastic bias for the rotating component 10 towards the surgical instrument 200 with increased deformation when the drive-input interface 50 is coupled with the rotating component 10, a first detection component 30 configured to detect the position of the rotating component 10 in the axial direction of the rotating component 10.

By adopting the first detection component 30 in the power apparatus, the detecting method includes: determining whether the drive-input interface 50 is coupled with the rotating component 10 based on the detection result from the first detection component 30. The drive-input interface 50 is configured to compress the axial biasing component 20 when coupled with the rotating component 10 and provide torque for the surgical instrument 200. Specifically, when the detected portion of the rotating component 10 is located at a position further from the surgical instrument 200 than a preset position, it is determined that the drive-input interface 50 is not coupled with the rotating component 10; otherwise, when the rotating component 10 is located at the preset position A0, it is determined that the drive-input interface 50 is coupled with the rotating component 10.

When the surgical robot does not comprise the adapter assembly 300, the first input disk 51 and the rotating component 10 are coupled by the engagement of concave and convex features (C1, C2, and F1, F2) on end surfaces facing each other. The drive-input interface 50 includes a first input disk 51 provided on the surgical instrument 200, and the first input disk 51 and the rotating component 10 are coupled by the engagement of concave and convex features on end surfaces facing each other. After the surgical instrument 200 is mounted to the power apparatus 100, the detecting method includes: determining whether the first input disk 51 is coupled with the rotating component 10 based on the detection result from the first detection component 30. When the detected portion of the rotating component 10 is located at a position further from the surgical instrument 200 than the preset position A0, it is determined that the drive-input interface 50 is not coupled with the coupling end 10S of the rotating component 10.

When the surgical robot includes the adapter assembly 300, and the adapter assembly 300 is part of the power apparatus 100, after the adapter assembly 300 is mounted to the power apparatus 100, the detecting method includes: determining whether the second input disk 52 is coupled with the rotating component 10 based on the detection result from the first detection component 30; after the second input disk 52 is coupled with the rotating component 10 and the surgical instrument 200 is mounted to the adapter assembly 300, determining whether the first input disk 51 is coupled with the second input disk 52 based on the detection result from the first detection component 30.

In one embodiment, the first detection component 30 determines the coupling state of the drive-input interface 50 by detecting whether the detected portion 101 of the rotating component 10 is sensed. Under this circumstance, the detecting method includes: when the first detection component 30 senses the detected portion 101 of the rotating component 10, it is determined that the drive-input interface 50 is not coupled with the rotating component 10; when the first detection component 30 does not detect the detected portion 101 of the rotating component 10, it is determined that the drive-input interface 50 is coupled with the rotating component 10.

In one embodiment, the first detection component 30 determines the coupling state of the drive-input interface 50 by detecting the distance between it and the detected portion 101. Under this circumstance, the detecting method includes: when the first detection component 30 detects that the distance between it and the rotating component 10 is less than a preset distance, it is determined that the drive-input interface 50 is not coupled with the rotating component 10; otherwise, it is determined that the drive-input interface 50 is coupled with the rotating component 10.

In one embodiment, the first detection component 30 determines the coupling state of the drive-input interface 50 by detecting the deformation of the axial biasing component 20. Under this circumstance, the detecting method includes: when the first detection component 30 detects that the deformation of the axial biasing component 20 is less than or greater than a preset value, it is determined that the drive-input interface 50 is not coupled with the rotating component 10; otherwise, when the first detection component 30 detects that the deformation of the axial biasing component 20 is equal to the preset value, it is determined that the drive-input interface 50 is coupled with the rotating component 10.

In one embodiment, the power apparatus 100 further comprises a first signal terminal P1 provided on the first housing 100a, which can be conductively connected to the signal terminal on the drive-input interface 50, thereby realizing the transmission of electrical signals. The detecting method includes: determining that the drive-input interface 50 is mounted to the power apparatus based on the signal indicating conduction of the first signal terminal P1 and the signal terminal of the drive-input interface 50; otherwise, it is determined that the drive-input interface 50 is not mounted or the mounting has failed.

In a power apparatus 100 without an adapter assembly 300, when a surgical instrument 200 is mounted to the first housing 100a of the power apparatus 100, the third signal terminal P3 of the adapter assembly 300 is conductively connected to the first signal terminal P1. The detecting method includes: determining that the surgical instrument 200 is mounted to the power apparatus based on the signal indicating conduction of the first signal terminal P1 and the third signal terminal P3.

In one embodiment, the power apparatus 100 includes an adapter assembly 300 configured to transmit torque between the rotating component 10 and the surgical instrument 200. The adapter assembly 300 has a second signal terminal P2, and the surgical instrument 200 has a third signal terminal P3. The second signal terminal P2 is capable of being electrically connected to the first signal terminal P1 of the first housing 100a the third signal terminal P3 of the surgical instrument 200. The detecting method includes: determining that the adapter assembly 300 is mounted to the first housing 100a based on the signal indicating conduction of the second signal terminal P2 and the first signal terminal P1; after the adapter assembly 300 is mounted to the first housing 100a, determining that the surgical instrument 200 is mounted to the adapter assembly 300 based on the signal indicating conduction of the third signal terminal P3 of the surgical instrument 200 and the first signal terminal P1.

In one embodiment, the power apparatus 100 further comprises an indication device 301 provided on the adapter assembly 300 and electrically connected to the second signal terminal P2. Thus, the second signal terminal P2 can conduct with the first signal terminal P1 after the adapter assembly 300 is mounted to the first housing 100a, and can conduct with the third signal terminal P3 after the surgical instrument 200 is mounted to the adapter assembly 300. The detecting method includes: switching the indication state of the indication device based on the detection result from the first detection component 30 after the adapter assembly 300 is mounted to the first housing 100a, and/or after the surgical instrument 200 is mounted to the adapter assembly 300. For example, when the indication device 301 is an indicator light, it turns off when the second input disk 52 of the adapter assembly 300 is detected to be coupled with the rotating component 10, and it also turns off when the first input disk 51 of the surgical instrument 200 is coupled with the second input disk 52. In other embodiments, the indication device 301 can also be a loudspeaker or a display device, etc. The indication information of the indication device 301 can also be issued in other ways, such as displaying through the main operation console 1 or an independent imaging device (such as an imaging cart).

### Embodiment 3

Referring to FIG. 11, the present embodiment provides an engaging method for a power apparatus of a surgical robot, comprising:

S01: Mounting the drive-input interface 50 to the coupling end 10S of the rotating component 10 of the power apparatus, wherein the rotating component 10 is elastically abutted against the drive-input interface 50 under the action of the axial biasing component 20, and the drive-input interface 50 can provide torque for the surgical instrument 200;

S02: Detecting the position of the rotating component 10 in the axial direction of the rotating component 10;

S03: When the detection result indicates that the detected portion of the rotating component 10 is located at a position further from the surgical instrument 200 than a preset position, rotating the rotating component 10 in a preset manner (such as forward and/or reverse) to move the rotating component 10 along the axial direction of the rotating component 10 to the preset position A0; when the detection result indicates that the rotating component 10 is located at the preset position A0 along the axial direction of the rotating component 10, it is determined that the drive-input interface 50 is successfully engaged with the rotating component 10.

When the surgical robot does not include an adapter assembly 300, the first input disk 51 and the rotating component 10 are coupled through the engagement of concave and convex features (C1, C2, and F1, F2) on end surfaces facing each other. The drive-input interface 50 includes the first input disk 51 provided on the surgical instrument 200, which is coupled with the rotating component 10 through the engagement of concave and convex features on end surfaces facing each other. During the engagement of the surgical instrument and the power apparatus, the surgical instrument 200 is first mounted to the first housing 100a of the power apparatus 100. Then, the position of the rotating component 10 in the axial direction of the rotating component 10 is detected. If the rotating component 10 is not at the preset position A0, it is rotated in a preset manner, such as forward and/or reverse, until the rotating component 10 moves along the axial direction of the rotating component 10 to the preset position A0, completing the coupling of the first input disk 51 and the rotating component 10.

When the surgical robot includes an adapter assembly 300, which serves as part of the power apparatus 100, the adapter assembly 300 includes a second input disk 52 with an axial and a circumferential degrees of freedom. The second input disk 52 is coupled with the rotating component 10 through the engagement of concave and convex features on end surfaces facing each other. The first input disk 51 provided on the surgical instrument 200 is coupled with the second input disk 52 through the engagement of concave and convex features on end surfaces facing each other. Under this circumstance, the engagement method involves two mounting processes for the adapter assembly 300 and the surgical instrument 200. First, the adapter assembly 300 is mounted to the first housing 100a of the power apparatus 100. Then, the position of the rotating component 10 in the axial direction of the rotating component 10 is detected. If the detected portion of the rotating component 10 is located at a position further from the surgical instrument than the preset position, the rotating component 10 is rotated in a preset first manner, such as forward and/or reverse, to move the rotating component 10 along the axial direction of the rotating component 10 to the preset position A0, completing the mounting of the adapter assembly 300 and the coupling of the second input disk 52. Subsequently, the surgical instrument 200 is mounted to the second housing 300a of the adapter assembly 300. The position of the rotating component 10 in the axial direction of the rotating component 10 is then detected. If the rotating component 10 is not at the preset position A0, and the detected portion of the rotating component 10 is located at a position further from the surgical instrument than the preset position A0 due to the pressing force from the second input disk 52, the rotating component 10 is rotated in a preset second manner, such as forward and/or reverse, to move the rotating component 10 along the axial direction of the rotating component 10 to the preset position A0, completing the mounting of the surgical instrument 200 and the coupling of the first input disk 51.

Typically, when the power apparatus 100 is powered on, the motor M drives the rotating component 10 to be rotated to the zero position. A second detection component 60 is used to detect the zero position of the rotating component 10. A target portion T is provided on the rotating component 10. When the motor M drives the rotating component 10 to be rotated to the zero position, the target portion T is rotated to be aligned with the second detection component 60. Before mounting the drive-input interface 50 to the coupling end 10S of the rotating component 10 of the power apparatus 100, the rotating component 10 can be rotated until the target portion T is detected by the second detection component 60, thus returning the rotating component 10 to the zero position for convenience of subsequent operations.

The target portion T can be a through-hole in the axial direction of the rotating component 10. The second detection component 60 includes a transmitting end 61 and a receiving end 62 disposed on two sides of the target portion T along the axial direction of the rotating component 10. When the signal emitted by the transmitting end 61 is received by the receiving end 62, it is indicated that the target portion T is rotated to be between the transmitting end 61 and the receiving end 62; otherwise, it is indicated that the target portion T is not directly opposite to the second detection component 60. The target portion T can be disposed on the annular detected portion 101, and the transmitting end 61 and the receiving end 62 are located on two sides of the detected portion 101 along the axial direction of the rotating component 10, clamping the detected portion 101 therebetween. An axial movement gap 600 reserved for the detected portion 101 is formed between the transmitting end 61 and the receiving end 62. The rotation of the rotating component 10 to the zero position is specifically indicated by the detection of the target portion T by the second detection component 60.

In other embodiments, the target portion T can be another traceable identifier. For example, the target portion T can be a magnet, and the second detection component 60 can be a Hall sensor. By utilizing the Hall sensor to detect changes in the magnetic field generated by the magnet nearby, the rotation angle of the rotating component 10 can be identified, thereby determining the zero position.

In addition to the rotating component 10 needing to return to the zero position when the power apparatus 100 is powered on, when the adapter assembly 300 is mounted to the first housing 100a and the second input disk 52 is successfully coupled with the rotating component 10, the rotating component 10 may also need to return to the zero position. The rotating component 10 returning to the zero position before mounting the surgical instrument 200 allows the surgical instrument 200 to be directly drivingly coupled with the adapter assembly 300 after being mounted to the adapter assembly 300, or requires only minor adjustment of the rotation angle of the rotating component 10 to be coupled with the corresponding first input disk 51, which can improve the engagement efficiency of the surgical instrument. Specifically, when the adapter assembly 300 is mounted to the first housing 100a, and the first detection component 30 detects that the second input disk 52 of the adapter assembly 300 is drivingly coupled with the rotating component 10, the rotating component 10 is controlled to be rotated in the direction towards the zero position, allowing the target portion T to be detected by the second detection component 60. In some embodiments, if the rotating component 10 is not returned to the zero position before mounting the surgical instrument 200, the first input disk 51 needs to be aligned and coupled with the corresponding second input disk 52 after mounting the surgical instrument 200, which may take more time.

The rotating component 10 includes a first rotating component 10A and a second rotating component 10B. Each rotating component 10 corresponds to one drive-input interface 50, one first detection component 30, and one second detection component 60. The first rotating component 10A includes a rotating component 10 configured to drive the end effector of the surgical instrument 200 to perform non-self-rotation movements such as pitch, yaw, opening, and closing. The second rotating component 10B comprises a rotating component 10 configured to drive the end effector of the surgical instrument 200 to perform self-rotation movements. During the engagement of the drive-input interface 50, when the surgical instrument 200 is mounted to the adapter assembly 300 and the first detection component 30 detects that the drive-input interface 50 is coupled with the first rotating component 10A, the first rotating component 10A can be rotated to the zero position. If the target portion T is detected by the second detection component 60, the rotation of the first rotating component 10A can be stopped; otherwise, the first rotating component 10A is controlled to be rotated until the target portion T is detected by the second detection component 60, bringing the first rotating component 10A back to the zero position. However, after the surgical instrument 200 is mounted to the adapter assembly 300 and the first detection component 30 detects that the drive-input interface 50 is coupled with the second rotating component 10B, if the target portion T is in a preset angular range α, the rotation of the second rotating component 10B is stopped; otherwise, the second rotating component 10B is controlled to be rotated until the target portion T is in the preset angular range α. The preset angular range α can be defined based on data from he encoder of the motor M, and whether the target portion T is in the preset angular range α can be calculated based on the rotation angle of the motor M.

When the surgical robot includes an adapter assembly 300, the second input disk 52 of the adapter assembly 300 has an axial and a circumferential degrees of freedom. After the adapter assembly 300 is mounted to the first housing 100a, if the second input disk 52 is not coupled with the rotating component 10, the rotating component 10 is controlled to be rotated in a preset first manner. When the rotating component 10 is rotated to be coupled with the second input disk 52, the rotation of the rotating component 10 in the preset first manner is interrupted. The preset first manner can comprise scanning by rotation in forward and reverse directions for M times. The rotation from the initial position to the extreme position in the forward direction and then returning to the initial position is referred to as scanning. One rotation from the initial position to the extreme position in the forward direction or one rotation from the extreme position to the initial position one time is referred to as a unidirectional scanning. The angle of unidirectional scanning of the rotating component 10 is equal to or less than a first angular threshold a. Subsequently, when the first input disk 51 is not coupled with the second input disk 52, the rotating component 10 is controlled to be rotated in a preset second manner. When the rotating component 10 is rotated to be coupled with the first input disk 51, the rotation of the rotating component 10 in the preset second manner is interrupted. The preset second manner can be, for example, forward and reverse rotation for scanning, such as scanning for N times. The preset second manner can comprise scanning by rotation in forward and reverse directions for N times, wherein angle of unidirectional scanning of the rotating component 10 is equal to or less than a second angle threshold b. M and N are both equal to or greater than 1 The first angular threshold a is greater than the second angular threshold b to avoid causing uncontrolled accidental motion of the end effector in the wound.

It can be understood that in some embodiments, the process of combining the surgical instrument with the power apparatus can be as follows. After detecting that the adapter assembly 300 is mounted to the first housing 100a, regardless of whether the second input disk 52 is coupled with the rotating component 10, the rotating component 10 is controlled to be rotated forward and reverse for scanning, and the angle of unidirectional scanning of the rotating component 10 is equal to or less than the first angular threshold a. After detecting that the surgical instrument 200 is mounted to the adapter assembly 300, regardless of whether the first input disk 51 is coupled with the second input disk 52, the rotating component 10 is controlled to be rotated forward and reverse for scanning, and the angle of unidirectional scanning of the rotating component 10 is equal to or less than the second angular threshold b, which is also less than the first angular threshold a.

Furthermore, in one embodiment, after the surgical instrument 200 is mounted to the adapter assembly 300 and drivingly engaged with the adapter assembly 300, the first rotating component 10A needs to be rotated to the zero position, and the process of combining the surgical instrument with the power apparatus can be as follows. When the first rotating component 10A is coupled with one second input disk 52, and the corresponding first input disk 51 is mounted to the corresponding second input disk 52 but not coupled with the first rotating component 10A, the first rotating component 10A is controlled to be rotated in a preset third manner. During this process, once the first detection component 30 detects that the first input disk 51 is coupled with the second input disk 52, the rotation of the first rotating component 10A in the preset third manner is immediately interrupted, and the rotating component 10 is controlled to be rotated towards the zero position. The rotation of the first rotating component 10A is stopped when the target portion T is detected by the second detection component 60. When the second rotating component 10B is coupled with its corresponding second input disk 52, and the corresponding first input disk 51 is mounted to the corresponding second input disk 52 but not coupled with the second rotating component 10B, the second rotating component 10B is controlled to be rotated in a preset fourth manner. When the second rotating component 10B is rotated to be coupled with the corresponding first input disk 51, the rotation of the second rotating component 10B in the preset fourth manner is interrupted, and the second rotating component 10B is controlled to be rotated until the target portion T is in the preset angular range α. That is, once the first detection component 30 detects that the first input disk 51 is coupled with the second input disk 52, regardless of whether the rotating component 10 is about to be rotated forward or reverse, the rotating component 10 is directly rotated towards the zero position until the target portion T is detected by the second detection component 60. The orientation of the zero position can be determined in conjunction with an encoder when the power apparatus 100 is powered on. This allows the rotating component 10 to return to the zero position in the shortest time and rotation path after the first input disk 51 is coupled with the second input disk 52 without performing unnecessary movements or waiting for the rotating component 10 to finish scanning a certain angular threshold, thereby improving mounting efficiency and safety.

In some embodiments, when the surgical instrument 200 is mounted to the adapter assembly 300 and the first detection component 30 detects that the first input disk 51 is coupled with the first rotating component 10A, the first rotating component 10A immediately stops rotating and does not return to the zero position. The process of combining the surgical instrument with the power apparatus can be as follows: when the first rotating component 10A is coupled with the second input disk 52, and the corresponding first input disk 51 is mounted to the corresponding second input disk 52 but not coupled with the first rotating component 10A, the first rotating component 10A is controlled to be rotated in a preset third manner. When the first rotating component 10A is rotated to be coupled with the corresponding first input disk 51, the rotation of the first rotating component 10A in the preset third manner is interrupted, and rotation of the first rotating component 10A is stopped without controlling the rotating component 10 to be rotated towards the zero position. When the second rotating component 10B is coupled with its corresponding second input disk 52, and the corresponding first input disk 51 is mounted to the corresponding second input disk 52 but not coupled with the second rotating component 10B, the second rotating component 10B is controlled to be rotated in a preset fourth mode. When the second rotating component 10B is rotated to be coupled with the corresponding first input disk 51, the rotation of the second rotating component 10B in the preset fourth mode is interrupted and the target portion T of the second rotating component 10B is controlled to be rotated in a preset angular range α. That is, once the first detection component 30 detects that the first input disk 51 is coupled with the second input disk 52, regardless of whether the rotating component 10 is about to rotate forward or reverse, rotation is directly stops rotating and the rotating component 10 waits for the next master-slave alignment command. When all drive-input interfaces 50 of the surgical instrument 200 are coupled with the corresponding rotating components 10 of the power apparatus and the target portion T of the second rotating component 10B is in the preset angular range α, the next master-slave alignment action can be performed to align the posture of the operating part 1a with the posture of the slave device 2.

Referring to FIG. 6B, additionally, considering that after the adapter assembly 300 is mounted to the first housing 100a, even if the rotating component 10 is controlled to be rotated repeatedly in forward and reverse directions for M times, the second input disk 52 still cannot be coupled with the rotating component 10, or after the rotating component 10 is controlled to be rotated repeatedly in forward and reverse directions for N times, the first input disk 51 still cannot be coupled with the second input disk 52, the adapter assembly 300 of the present embodiment further comprises an axial vibration component 302, which partially extends into the accommodation cavity 3011. When the second input disk 52 cannot be coupled with the rotating component 10, or the first input disk 51 cannot be coupled with the second input disk 52, the axial vibration component 302 can be controlled to vibrate the second input disk 52 along the axial direction of the adapter assembly 300 until the corresponding coupling features being engaged with each other. For example, the axial vibration component 302 can be a linear motor, fixed on the second housing 300a, and electrically connected to the controller 40 via the second signal terminal P2.

For example, an annular recess 520 can be formed on the outer circumferential surface of each second input disk 52. Part of the axial vibration component 302 is disposed in the second housing 300a, while another part extends into the recess 520. When the second input disk 52 is coupled with the rotating component 10 and the first input disk 51, the rotating component 10 is at a preset position A0, and the axial vibration component 302 may not contact the recess 520, thus not affecting the rotation of the second input disk 52. When the rotating component 10 is not at the preset position A0, the axial vibration component 302 drives the second input disk 52 to vibrate slightly in the axial direction and cooperates with the forward and reverse rotations of the rotating component 10, so that the rotating component 10, the second input disk 52, and the first input disk 51 can be coupled. Therefore, the process of combining the surgical instrument with the power apparatus can be as follows. when the adapter assembly 300 is mounted and the first coupling features C1, C2 are not aligned with the second coupling features D1, D2, the rotating component 10 is controlled to be rotated repeatedly in forward and reverse directions in to a preset manner the axial vibration component 302 is activated to axially vibrate the rotating component 10 relative to the second input disk 52, so as to align the first coupling features C1 and C2 with the second coupling features D1 and D2. Similarly, when the surgical instrument 200 is mounted and the third coupling features E1, E2 are not aligned with the fourth coupling features F1, F2, the rotating component 10 is controlled to be rotated repeatedly in forward and reverse directions in a preset manner and the axial vibration component 302 is activated, so that the second input disk 52 is rotated and axially vibrated relative to the first input disk 51, aligning the third coupling features E1 and E2 with the fourth coupling features F1 and F2.

### Embodiment 4

The present embodiment provides a computer-readable storage medium storing multiple instructions, which are suitable for being loaded and executed by at least one processor to perform the detecting method for the engagement state of the power apparatus of a surgical robot and/or the method for engaging the power apparatus of a surgical robot described above. The computer-readable storage medium is part of a bite force control system. In some embodiments, the processor can be a Central Processing Unit (CPU), a controller, a microcontroller, a microprocessor, or other data processing chips. The processor is usually used to control the overall operation of a computing device. In the present embodiment, the processor is used to run program codes stored in the storage medium or process data.

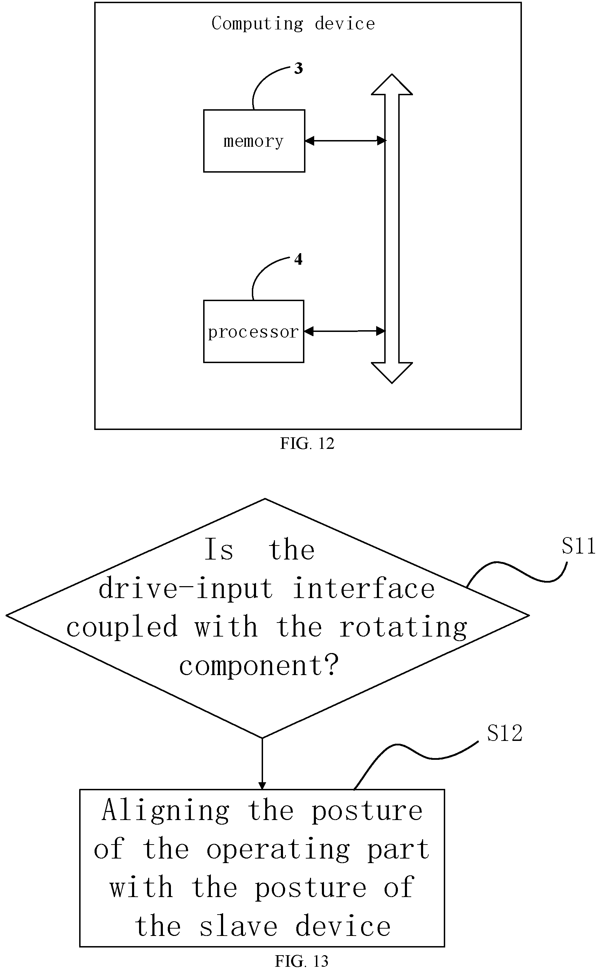

Referring to FIG. 12, the present embodiment provides a computing device, which includes a memory 3 and a processor 4. The memory 3 can be above-mentioned computer-readable storage medium, which stores multiple instructions suitable for being loaded and executed by at least one processor 4 to perform the detecting method and/or the engagement method described above.

### Embodiment 5

Referring to FIG. 13, the present embodiment provides a control method for a surgical robot, which includes:

S11: Determining whether the drive-input interface 50 is coupled with the rotating component 10 based on the detection result from the first detection component 30;

S12: Aligning the posture of the operating part 1a with the posture of the slave device after all drive-input interfaces 50 of the surgical instrument 200 are coupled with the rotating components 10 of the power apparatus. The present posture alignment step can be executed by the controller 40 or another controller.

The rotating component 10 includes a first rotating component 10A and a second rotating component 10B each corresponding to a second input disk 52 and a first input disk 51. To facilitate the next master-slave alignment procedure, before aligning the posture of the operating part 1a with the posture of the slave device 2 in step S12 and after all drive-input interfaces 50 of the surgical instrument 200 are coupled with the rotating components 10 of the power apparatus, it is necessary to detect whether the target part T of the second rotating component 10B is rotated to be in a preset angular range α. When the target part T of the second rotating component 10B is rotated to in the preset angular range α, the posture of the operating part is aligned with the posture of the slave device; otherwise, the posture alignment step is not executed. In addition, the control method further comprises the step of controlling the combination of the power apparatus and the surgical instrument, which has been described in detail in Embodiment 3 above.

It can be understood that the basis for determining whether the drive-input interface 50 is coupled with the rotating component 10 in step S11 of the present embodiment includes but is not limited to the detection of the first detection component 30. The detection result from the first detection component 30 can also be replaced by other methods. For example, it can be determined based on the resistance torque between the drive-input interface 50 and the rotating component 10. When the resistance torque between them reaches a preset threshold, it is considered that they are coupled; otherwise, it is determined that they are not coupled. Alternatively, it can also be determined based on the current of the motor M. When the current of the motor M reaches a preset current value, it is considered that they are coupled; otherwise, it is judged that they are not coupled. Another method is to determine based on the spacing between the first input disk 51 of the surgical instrument 200 and the rotating component 10. When the spacing between them is less than a preset distance threshold, it is determined that they are coupled; otherwise, it is determined that they are not coupled.

Through the description of the above embodiments, it is clear to those skilled in the art that the method of the above embodiments can be implemented by software plus a necessary general hardware platform, or of course, by hardware. However, in many cases, the former is a better implementation method. Based on this understanding, the technical solution of the present disclosure, or the part that contributes to the existing technology, can be embodied in the form of a software product. This computer software product is stored in a storage medium (such as ROM/RAM, magnetic disk, optical disk) and includes several instructions for enabling a terminal (which can be a mobile phone, computer, server, air conditioner, network device, etc.) to execute the methods described in the embodiments of the present disclosure.

In the description of this specification, the terms "an embodiment", "some embodiments", "example", "specific example", or "some examples" are used to indicate that the specific features, structures, materials, or characteristics described in connection with the embodiment or example are included in at least one embodiment or example of the present invention. In this specification, the illustrative expressions of the above terms do not necessarily refer to the same embodiment or example. Furthermore, the described specific features, structures, materials, or characteristics may be combined in any suitable manner in one or more embodiments or examples. Additionally, without contradiction, those skilled in the art can combine and integrate different embodiments or examples described in this specification, as well as the features of different embodiments or example.

The above-mentioned embodiments only express several implementation modes of the present disclosure, and the description is relatively specific and detailed, but they cannot be understood as limiting the scope of the patent application. It should be noted that for one skilled in the art, without departing from the concept of the present disclosure, several modifications and improvements can be made, and these all belong to the protection scope of the present disclosure. Therefore, the protection scope of the patent of the present disclosure should be subject to the appended claims.

## Claims

1. A power apparatus of a surgical robot, comprising:
a first housing (100a);
a rotating component (10) rotatably provided on the first housing (100a), comprising a target portion (T) and an axial coupling end (10S), wherein the coupling end (10S) is configured to be coupled with a drive-input interface (50) providing torque for a surgical instrument (200);
an axial biasing component (20) configured to provide an elastic bias towards the surgical instrument (200) for the rotating component (10);
a first detection component (30) configured to detect a position of the rotating component (10) in an axial direction of the rotating component (10);
a second detection component (60) configured to be aligned with the target portion (T) when the rotating component (10) is rotated to a zero position; and
a controller (40) configured to:
rotate the rotating component (10) until the target portion (T) is detected by the second detection component (60) before mounting the drive-input interface (50); and
when a detected portion of the rotating component (10) is located at a position further from the surgical instrument (200) than a preset position, rotate the rotating component (10) in a preset manner so as to move the rotating component (10) along the axial direction of the rotating component (10) to the preset position.

2. The power apparatus of claim 1, wherein the drive-input interface (50) comprises a first input disk (51) provided on the surgical instrument (200), and the first input disk (51) and the rotating component (10) are coupled by engagement of concave and convex features on end surfaces facing each other;
the controller (40) is configured to:
determine whether the first input disk (51) is coupled with the rotating component (10) based on a detection result from the first detection component (30) after the surgical instrument (200) is mounted to the power apparatus.

3. The power apparatus of claim 1, further comprising an adapter assembly (300), wherein the adapter assembly (300) comprises a second housing (300a) detachably mounted to the first housing (100a) and a second input disk (52) rotatably provided on the second housing (300a);
the drive-input interface (50) comprises the second input disk (52), and the second input disk (52) is capable of being coupled with the rotating component (10) by engagement of concave and convex features on end surfaces facing each other to transmit torque between the rotating component (10) and the surgical instrument (200);
the controller (40) is configured to:
determine whether the second input disk (52) is coupled with the rotating component (10) based on a detection result from the first detection component (30) after the second housing (300a) is mounted to the power apparatus.

4. The power apparatus of claim 3, wherein the second input disk (52) and the first input disk (51) provided on the surgical instrument (200) are coupled by engagement of concave and convex features on end surfaces facing each other, and the second input disk (52) has a freedom along an axial direction of the second housing (300a);
the controller (40) is configured to:
determine whether the first input disk (51) is coupled with the second input disk (52) based on a detection result from the first detection component (30) after the second input disk (52) is coupled with the rotating component (10) and the surgical instrument (200) is mounted to the power apparatus.

5. The power apparatus of claim 1, wherein a detection end of the first detection component (30) is provided on a side of the rotating component (10) that is further from the surgical instrument (200), and there is a gap along an axial direction of the rotating component (10) between the detection end of the first detection component (30) and the rotating component (10).

6. The power apparatus of claim 1, wherein the first detection component (30) is configured to detect whether the detected portion of the rotating component (10) is sensed; the controller (40) is configured to: determine that the drive-input interface (50) is not coupled with the rotating component (10) when the first detection component (30) senses the detected portion of the rotating component (10).

7. The power apparatus of claim 1, wherein the first detection component (30) is configured to detect a distance between the first detection component (30) and the detected portion (101); the controller (40) is configured to: determine that the drive-input interface (50) is not coupled with the rotating component (10) when the first detection component (30) detects that the distance between the first detection component (30) and the rotating component (10) is less than a preset distance.

8. The power apparatus of claim 1, further comprising an adapter assembly (300) detachably mounted to the first housing (100a), wherein the adapter assembly (300) is configured to transmit torque between the rotating component (10) and the surgical instrument (200);
the controller (40) is configured to:
control the rotating component (10) to be rotated until the target portion (T) is detected by the second detection component (60) when the adapter assembly (300) is mounted to the first housing (100a) and the first detection component (30) detects that the adapter assembly (300) is drivingly engaged with the rotating component (10).

9. The power apparatus of claim 1, further comprising an adapter assembly (300), wherein the adapter assembly (300) comprises a second input disk (52), and the surgical instrument (200) comprises a first input disk (51) coupled with the rotating component (10) via the second input disk (52);
the controller (40) is configured to:
control the rotating component (10) to be rotated in a preset first manner when the second input disk (52) is mounted to the rotating component (10) and not coupled with the rotating component (10), and interrupt rotation of the rotating component (10) in the preset first manner when the rotating component (10) is rotated to be coupled with the second input disk (52); and/or
control the rotating component (10) to be rotated in a preset second manner when the rotating component (10) is coupled with the second input disk (52) and the first input disk (51) is mounted to the second input disk (52) and not coupled with the rotating component (10), and interrupt rotation of the rotating component (10) in the preset second manner when the rotating component (10) is rotated to be coupled with the first input disk (51).

10. The power apparatus of claim 9, wherein the rotating component (10) comprises a first rotating component (10A) and a second rotating component (10B) each corresponding to one of the second input disk (52) and one of the first input disk (51);
the controller (40) is configured to:
control the first rotating component (10A) to be rotated in a preset third manner when the first rotating component (10A) is coupled with the second input disk (52) corresponding to the first rotating component (10A) and the first input disk (51) corresponding to the first rotating component (10A) is mounted to the second input disk (52) corresponding to the first rotating component (10A) and not coupled with the first rotating component (10A), interrupt the rotation of the first rotating component (10A) in the preset third manner when the first rotating component (10A) is rotated to be coupled with the first input disk (51) corresponding to the first rotating component (10A), and control the first rotating component (10A) to be rotated until the target portion (T) is detected by the second detection component (60); and
control the second rotating component (10B) to be rotated in a preset fourth manner when the second rotating component (10B) is coupled with the second input disk (52) corresponding to the second rotating component (10B) and the first input disk (51) corresponding to the second rotating component (10B) is mounted to the second input disk (52) corresponding to the second rotating component (10B) and not coupled with the second rotating component (10B), interrupt the rotation of the second rotating component (10B) in the preset fourth manner when the second rotating component (10B) is rotated to be coupled with the first input disk (51) corresponding to the second rotating component (10B), and control the second rotating component (10B) to be rotated until the target portion (T) is in a preset angular range (α).

11. The power apparatus of claim 9, wherein the rotating component (10) comprises a first rotating component (10A) and a second rotating component (10B) each corresponding to one of the second input disk (52) and one of the first input disk (51);
the controller (40) is configured to:
control the first rotating component (10A) to be rotated in a preset third manner when the first rotating component (10A) is coupled with the second input disk (52) corresponding to the first rotating component (10A) and the first input disk (51) corresponding to the first rotating component (10A) is mounted to the second input disk (52) corresponding to the first rotating component (10A) and not coupled with the first rotating component (10A), interrupt the rotation of the first rotating component (10A) in the preset third manner when the first rotating component (10A) is rotated to be coupled with the first input disk (51) corresponding to the first rotating component (10A), and stop rotating the first rotating component (10A); and
control the second rotating component (10B) to be rotated in a preset fourth manner when the second rotating component (10B) is coupled with the second input disk (52) corresponding to the second rotating component (10B) and the first input disk (51) corresponding to the second rotating component (10B) is mounted to the second input disk (52) corresponding to the second rotating component (10B) and not coupled with the second rotating component (10B), interrupt the rotation of the second rotating component (10B) in the preset fourth manner when the second rotating component (10B) is rotated to be coupled with the first input disk (51) corresponding to the second rotating component (10B), and control the second rotating component (10B) to be rotated until the target portion (T) is in a preset angular range (α).

12. The power apparatus of claim 9, wherein the preset first manner and the preset second manner both comprise scanning by rotation in forward and reverse directions, an angle of unidirectional scanning in the first manner is equal to or less than a first angular threshold (a), the angle of unidirectional scanning in the second manner is equal to or less than a second angular threshold (b), and the first angular threshold (a) is greater than the second angular threshold (b).

13. The power apparatus of claim 3, further comprising an indication device (301), wherein the first housing (100a) is provided with a first signal terminal (P1), the adapter assembly (300) comprises a second signal terminal (P2), the surgical instrument (200) comprises a third signal terminal (P3), the indication device (301) is provided on the adapter assembly (300) and electrically connected to the second signal terminal (P2);
the second signal terminal (P2) is capable of being conductively connected to the first signal terminal (P1) after the adapter assembly (300) is mounted to the first housing (100a), and is capable of being conductively connected to the third signal terminal (P3) after the surgical instrument (200) is mounted to the adapter assembly (300);
the controller (40) is configured to:
switch an indication state of the indication device according to the detection result from the first detection component (30) after the adapter assembly (300) is mounted to the first housing (100a) and/or after the surgical instrument (200) is mounted to the adapter assembly (300).

14. The power apparatus of claim 3, wherein an edge of a surface of the second input disk (52) having a concave or convex feature is protruded with multiple first protrusions (521), a surface of the second housing (300a) facing the surface of the second input disk (52) having the concave or convex feature is protruded with multiple second protrusions (3000), and the second protrusions (3000) are configured to restrict rotation of the second input disk (52) when each of the first protrusions (521) is embedded between two second protrusions (3000).

15. A surgical robot, comprising a surgical instrument (200) and the power apparatus of any one of claims 1 to 14, wherein the surgical instrument (200) is configured to perform corresponding actions driven by the power apparatus.

16. An engaging method for a power apparatus of a surgical robot, wherein the power apparatus comprises:
a rotating component (10) comprising an axial coupling end (10S);
an axial biasing component (20) configured to provide an elastic bias towards a surgical instrument (200) for the rotating component (10), wherein deformation of the axial biasing component (20) is increased when a drive-input interface (50) providing torque for the surgical instrument (200) is coupled with the rotating component (10);
a first detection component (30) configured to detect a position of the rotating component (10) in an axial direction of the rotating component (10);
the engaging method comprises:
detecting the position of the rotating component (10) in the axial direction of the rotating component (10) after the drive-input interface (50) is mounted the rotating component (10) of the power apparatus; and
rotating the rotating component (10) in a preset manner when a detected portion of the rotating component (10) is located at a position further from the surgical instrument (200) than a preset position so as to move the rotating component (10) along the axial direction of the rotating component (10) to the preset position.

17. The engaging method of claim 16, wherein the power apparatus further comprises a second detection component (60), and the rotating component (10) is provided with a target portion (T);
the engaging method comprises:
rotating the rotating component (10) until the target portion (T) is detected by the second detection component (60) before mounting the drive-input interface (50).

18. The engaging method of claim 16, wherein the drive-input interface (50) comprises a first input disk (51) provided on the surgical instrument (200), and the first input disk (51) and the rotating component (10) are coupled by engagement of concave and convex features on end surfaces facing each other.

19. The engaging method of claim 16, wherein the power apparatus further comprises an adapter assembly (300) comprising a second input disk (52) having an axial degree of freedom and a circumferential degree of freedom, the second input disk (52) is capable of being coupled with the rotating component (10) by engagement of concave and convex features on end surfaces facing each other, and the second input disk (52) is coupled with a first input disk (51) provided on the surgical instrument (200) by engagement of concave and convex features on end surfaces facing each other;
the engaging method comprises:
mounting the adapter assembly (300) to the power apparatus;
detecting the position of the rotating component (10) in the axial direction of the rotating component (10);
rotating the rotating component (10) in a preset first manner when the detected portion of the rotating component (10) is located at the position further from the surgical instrument (200) than the preset position so as to move the rotating component (10) along the axial direction of the rotating component (10) to the preset position;
mounting the surgical instrument (200) to the adapter assembly (300);
detecting the position of the rotating component (10) in the axial direction of the rotating component (10); and
rotating the rotating component (10) in a preset second manner when the detected portion of the rotating component (10) is located at the position further from the surgical instrument (200) than the preset position so as to move the rotating component (10) along the axial direction of the rotating component (10) to the preset position.

20. The engaging method of claim 19, wherein the power apparatus further comprises a second detection component (60), and the rotating component (10) is provided with a target portion (T);
the engaging method comprises:
controlling rotation of the rotating component (10) until the target portion (T) is detected by the second detection component (60) after the adapter assembly (300) is mounted to the power apparatus and the rotating component (10) is located in the preset position along the axial direction of the rotating component (10).

21. The engagement method of claim 19, further comprising:
after rotating the rotating component (10) in a preset first manner, interrupting the rotation of the rotating component (10) in the preset first manner when the rotating component (10) moves to the preset position along the axial direction of the rotating component (10); and/or
after rotating the rotating component (10) in a preset second manner, interrupting the rotation of the rotating component (10) in the preset second manner when the rotating component (10) moves to the preset position along the axial direction of the rotating component (10).

22. The engagement method of claim 21, wherein the power apparatus further comprises a second detection component (60), and the rotating component (10) is provided with a target portion (T);
the rotating component (10) comprises a first rotating component (10A) and a second rotating component (10B) each corresponding to one of the second input disk (52) and one of the first input disk (51);
the engagement method comprises:
controlling the first rotating component (10A) to be rotated in a preset third manner when the first rotating component (10A) is coupled with the second input disk (52) corresponding to the first rotating component (10A) and the first input disk (51) corresponding to the first rotating component (10A) is mounted to the second input disk (52) corresponding to the first rotating component (10A) and not coupled with the first rotating component (10A), interrupt the rotation of the first rotating component (10A) in the preset third manner when the first rotating component (10A) is rotated to be coupled with the first input disk (51) corresponding to the first rotating component (10A), and control the first rotating component (10A) to be rotated until the target portion (T) is detected by the second detection component (60); and
controlling the second rotating component (10B) to be rotated in a preset fourth manner when the second rotating component (10B) is coupled with the second input disk (52) corresponding to the second rotating component (10B) and the first input disk (51) corresponding to the second rotating component (10B) is mounted to the second input disk (52) corresponding to the second rotating component (10B) and not coupled with the second rotating component (10B), interrupt the rotation of the second rotating component (10B) in the preset fourth manner when the second rotating component (10B) is rotated to be coupled with the first input disk (51) corresponding to the second rotating component (10B), and control the second rotating component (10B) to be rotated until the target portion (T) is in a preset angular range (α).

23. The engagement method of claim 21, wherein the power apparatus further comprises a second detection component (60), and the rotating component (10) is provided with a target portion (T);
the rotating component (10) comprises a first rotating component (10A) and a second rotating component (10B) each corresponding to one of the second input disk (52) and one of the first input disk (51);
the engagement method comprises:
controlling the first rotating component (10A) to be rotated in a preset third manner when the first rotating component (10A) is coupled with the second input disk (52) corresponding to the first rotating component (10A) and the first input disk (51) corresponding to the first rotating component (10A) is mounted to the second input disk (52) corresponding to the first rotating component (10A) and not coupled with the first rotating component (10A), interrupt the rotation of the first rotating component (10A) in the preset third manner when the first rotating component (10A) is rotated to be coupled with the first input disk (51) corresponding to the first rotating component (10A), and stop rotating the first rotating component (10A); and
controlling the second rotating component (10B) to be rotated in a preset fourth manner when the second rotating component (10B) is coupled with the second input disk (52) corresponding to the second rotating component (10B) and the first input disk (51) corresponding to the second rotating component (10B) is mounted to the second input disk (52) corresponding to the second rotating component (10B) and not coupled with the second rotating component (10B), interrupt the rotation of the second rotating component (10B) in the preset fourth manner when the second rotating component (10B) is rotated to be coupled with the first input disk (51) corresponding to the second rotating component (10B), and control the second rotating component (10B) to be rotated until the target portion (T) is in a preset angular range (α).

24. A control method for a surgical robot comprising an operating part and a slave device, wherein the slave device comprises:
multiple rotating components (10) each comprising an axial coupling end (10S);
multiple axial biasing components (20) each configured to provide an elastic bias toward a surgical instrument (200) for each of the rotating components (10), wherein deformations of the axial biasing components (20) are increased when drive-input interfaces (50) providing torque for the surgical instrument (200) are coupled with the rotating components (10); and
multiple first detection components (30), each configured to detect a position of one of the rotating components (10) in an axial direction of the one of the rotating components (10);
the control method comprises:
determining whether the drive-input interfaces (50) are coupled with the rotating components (10) based on detection results from the first detection components (30); and
aligning a posture of the operating part with a posture of the slave device after all the drive-input interfaces (50) of the surgical instrument (200) are coupled with corresponding rotating components (10) of the power apparatus.

25. The control method of claim 24, wherein the rotating components (10) comprises a first rotating component (10A) and a second rotating component (10B) each corresponding to a second input disk (52) and a first input disk (51);
before aligning the posture of the operating part with the posture of the slave device and after all the drive-input interfaces (50) of the surgical instrument (200) are coupled with corresponding rotating components (10) of the power apparatus, the control method further comprises:
detecting whether the target portion (T) of each of the second rotating components (10B) is rotated to be a preset angular range (α); and
aligning the posture of the operating part with the posture of the slave device when the target portion (T) of each of the second rotating component (10B) is rotated to be in the preset angular range (α), otherwise, not performing the posture alignment step.
